(19) Europäisches Patentamt
European Patent Office
Office européen des brevets

(11) EP 3 533 419 A1

(12) EUROPEAN PATENT APPLICATION

(43) Date of publication:
04.09.2019 Bulletin 2019/36

(51) Int Cl.:
*A61F 2/68* (2006.01) *A61F 2/70* (2006.01)
*A61F 2/80* (2006.01)

(21) Application number: 19166878.9

(22) Date of filing: 02.09.2015

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 02.09.2014 US 201462044924 P

(62) Document number(s) of the earlier application(s) in accordance with Art. 76 EPC:
15838325.7 / 3 188 697

(71) Applicants:
- **The Ohio Willow Wood Company**
  **Mount Sterling, Ohio 43143 (US)**
- **Haynes, Michael L.**
  **Bexley, Ohio 43209 (US)**
- **Nixon, Derek**
  **Grove City, Ohio 43123 (US)**
- **Denune, Jeffrey A.**
  **Galloway, OH 43119 (US)**

(72) Inventors:
- **Haynes, Michael, L.**
  **Bexley, OH 43209 (US)**
- **Nixon, Derek**
  **Grove City, OH 43123 (US)**
- **Denune, Jeff**
  **Galloway, OH 43119 (US)**

(74) Representative: **Hutchison, Craig McGregor**
**Lawrie IP Limited**
**310 St Vincent Street**
**Glasgow G2 5RG (GB)**

Remarks:
This application was filed on 02-04-2019 as a divisional application to the application mentioned under INID code 62.

# (54) SYSTEMS FOR INTELLIGENT VACUUM SUSPENSION CONTROL

(57) According to the embodiments provided herein, a system for intelligent vacuum control can include a prosthetic socket, an electric vacuum pump, one or more sensors, a non-transient computer readable medium, and a processor. The stiffness data can correspond to a relationship between pressure variation of the internal pressure of the prosthetic socket and the relative socket motion of the prosthetic socket. The processor can execute program instructions to automatically compare the data indicative of the relative socket motion of the prosthetic socket and the stiffness data. The processor can execute the program instructions to automatically determine a vacuum suspension range based upon the comparison of the data indicative of the relative socket motion of the prosthetic socket and the stiffness data.

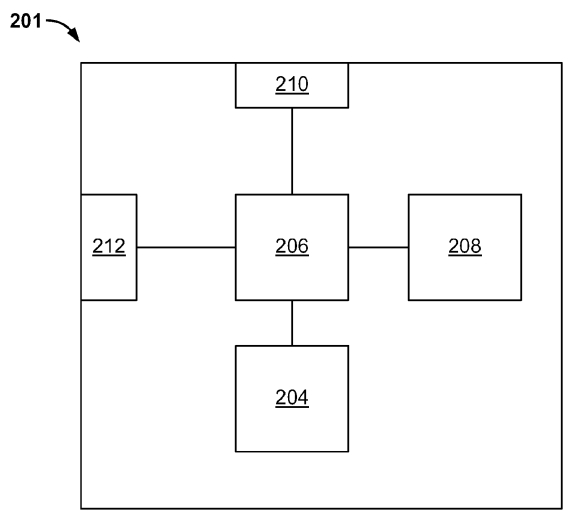


FIG. 2



Printed by Jouve, 75001 PARIS (FR)

## Description

CROSS-REFERENCE TO RELATED APPLICATIONS

This application claims priority to U.S. Provisional Patent Application No. 62/044,924 filed on September 2, 2014, which is entirely incorporated by reference herein.

TECHNICAL FIELD

The present disclosure generally relates to systems and methods for vacuum suspension control. Specifically, the present disclosure relates to systems and methods for intelligent vacuum suspension control of artificial limbs and prosthetic devices.

The United States government has rights in this invention pursuant to Contract No.VA11812C0038 with the VA Center for Innovation (VACI), an agency of the United States Department of Veterans Affairs.

BACKGROUND ART

Artificial limbs and prosthetic devices have been in use throughout history. Since its development, interfacing and suspending the prosthetic device has been an ongoing challenge. Various theories and anatomical constructs have been used over time in an evolving manner, and these have revealed a number of key factors in maximizing comfort and functional potential for users of prosthetic devices.

Surgical procedures used to perform limb amputations are an important factor. The size and shaping of a patient's residual limb is often important to the comfort the patient will later have with a prosthetic device. It is critical that the residual limb and prosthetic device interface tightly, couple, and distribute pressure evenly across the surface of the residual limb.

One system to suspend the artificial limb upon the user relies upon application of sub-atmospheric pressure (i.e. vacuum suspension). The use of sub-atmospheric pressure creates a linkage that provides excellent proprioceptive feedback and control for the artificial limb user. It also provides an excellent linkage between the user's limb and the prosthetic device.

Creating a reliable sub-atmospheric pressure chamber between the residual limb and prosthetic device has, however, proven to be a challenge. As new, airtight materials have evolved, along with airtight liners, the potential for creating a sub-atmospheric pressure within the prosthetic chamber (socket) has improved. Specifically, the patient's residual limb may be covered with a roll-on liner, which may help to protect the user's tissue from unwanted, isolated, high negative pressure values; and may provide cushioning for the tissue at the same time. The liner also helps to distribute the sub-atmospheric pressure applied to the user's limb in a more uniform manner. However, because a given patient may or may not utilize such a liner, "liner," "residual limb," "stump," "limb," and "leg" may all refer to a patient's residual limb with or without a liner, and will be used interchangeably herein when discussing a patient's residual limb, with or without a liner.

Referring now to **FIG. 1,** vacuum suspended prosthetic device **100** (also referred to herein as prosthetic device **100** and prosthesis **100**) may include socket **102** with an attached prosthetic limb (not shown) and electric vacuum pump **104a** or **104b** to evacuate air from within socket **102** to create a negative pressure (vacuum) between socket **102** and a user's residual limb (not shown), and thus suspend socket **102** from a user's residual limb. U.S. Pat. Nos. 7,914,586; 7,947,085; 8,016,892; as well as U.S. Publication Nos. 2008/0243266 and 2012/0004737 (The Ohio Willow Wood Company), all of which are incorporated herein by reference in their entireties, all describe vacuum suspension systems.

Vacuum suspended prosthetic devices are unique and customized to each user. As used herein, a user of a vacuum suspended prosthetic device may be called a "user," "patient," and "amputee." Because of the unique shape, geometry, and volume of each users residual limb, socket **102** may be customized for each user. Similarly, an amount of negative pressure provided by electric vacuum pump **104a, 104b** to suspend socket **102** to a user's residual limb may also be unique for each user.

A practitioner, as used herein "practitioner," "prosthetist," and "doctor," each used interchangeably, may consult numerous times with a prosthetic user over the course of the user's lifetime and lifetime of the prosthetic device to constantly adjust the prosthetic device to provide the best fit and function of the prosthetic device.

After customizing socket **102** to properly fit and accommodate the volume and shape of a user's residual limb, a consultation with a practitioner may include the practitioner adjusting vacuum suspension levels within socket **102** to find a suspension level that is both healthy and comfortable for a user's residual limb. For example, increasing vacuum suspension level can correspond to an increase in amplitude of negative pressure or vacuum. Likewise, decreasing vacuum suspension level can correspond to a decrease in amplitude of negative pressure or vacuum. A practitioner may have a user test a prosthetic device in different scenarios to determine a range of comfortable vacuum suspension levels without compromising the health of the user or function of the prosthetic device.

Historically, the solution to compensate for relative motion between the residual limb and the prosthetic socket in vacuum suspended prosthetic limbs was the application of higher vacuum suspension levels to increase suspension forces. However, higher vacuum suspension levels may negatively affect a user's residual limb by reducing circulation within the residual limb and/or causing pain to the residual limb during use. Further complications may arise in users with diabetic neuropathy who may have poor, or no feeling within their residual limbs. Accordingly, while a practitioner may be able to provide a good fit and suspension based on feedback from a user, there is still a need for improved vacuum suspension control.

Vacuum suspension technology has improved to include prosthetic sockets with adaptive vacuum systems that detect one or more activity levels of a patient and accordingly provide an appropriate amount of vacuum within the socket based on the current activity level of the patient.

The present application appreciates that adaptive vacuum suspension control between a prosthetic socket and a patient's residual limb may be a challenging endeavor.

## SUMMARY

In one embodiment, a system for intelligent vacuum control can include a prosthetic socket, an electric vacuum pump, one or more sensors, a non-transient computer readable medium, and a processor. The electric vacuum pump can create a sub-atmospheric pressure within the prosthetic socket. The one or more sensors can sense an internal pressure of the prosthetic socket and data indicative of relative socket motion of the prosthetic socket. The non-transient computer readable medium can include program instructions and stiffness data. The stiffness data can correspond to a relationship between pressure variation of the internal pressure of the prosthetic socket and the relative socket motion of the prosthetic socket. The processor can be operatively connected to the electric vacuum pump, the one or more sensors, and the non-transient computer readable medium. The processor can execute the program instructions to automatically compare the data indicative of the relative socket motion of the prosthetic socket and the stiffness data. The processor can execute the program instructions to automatically determine a vacuum suspension range based upon the comparison of the data indicative of the relative socket motion of the prosthetic socket and the stiffness data. The processor can execute the program instructions to automatically actuate the electric vacuum pump. The internal pressure of the prosthetic socket can be adjusted to be within the vacuum suspension range.

In another embodiment, a method for intelligent vacuum control can include collecting, at one or more suspension levels, motion data of a prosthetic socket, data indicative of internal pressure of the prosthetic socket and data indicative of relative socket motion of the prosthetic socket with one or more sensors coupled to a prosthetic device. An activity level associated with the data indicative of the internal pressure of the prosthetic socket and the data indicative of the relative socket motion of the prosthetic socket can be determined via the motion data of the prosthetic socket. An electric vacuum pump can be actuated automatically with a processor operatively coupled to the electric vacuum pump. The internal pressure of the prosthetic socket can be adjusted to be within each of the one or more suspension levels, while the motion data of the prosthetic socket, the data indicative of the internal pressure of the prosthetic socket and the data indicative of the relative socket motion of the prosthetic socket are collected.

In another embodiment, a system for intelligent vacuum control can include a prosthetic socket, an electric vacuum pump, a pressure sensor, a non-transient computer readable medium, and a processor. The electric vacuum pump can create a sub-atmospheric pressure within the prosthetic socket. The pressure sensor can sense an internal pressure of the prosthetic socket and provide a pressure waveform indicative of relative socket motion of the prosthetic socket. The non-transient computer readable medium can include program instructions. The processor can be operatively connected to the electric vacuum pump, the one or more sensors, and the non-transient computer readable medium. The processor can execute the program instructions to automatically detect one or more peaks in the pressure waveform. The processor can execute the program instructions to automatically quantify the relative socket motion based upon the one or more peaks detected in the pressure waveform. The processor can execute the program instructions to automatically compare a magnitude of the one or more peaks that are detected to an acceptable value. The processor can execute the program instructions to automatically rate a quality of suspension during a step based upon the magnitude of the one or more peaks and the acceptable value that are compared.

In another embodiment, a method for intelligent vacuum suspension of a prosthetic socket is provided, the method can include: determining at least one activity level threshold between two or more activity levels; determining at least one vacuum suspension range for one or more activity levels; determining at least one residual limb motion threshold; real time monitoring of motion of the prosthetic socket; real time monitoring of a vacuum suspension level; and real time monitoring of residual limb motion.

In another embodiment, a method of qualifying and quantifying one or more steps taken by a prosthetic leg device using an intelligent vacuum suspension system during a gait cycle is provided, the method can include the steps of: real time monitoring of an internal socket pressure during the gait cycle for a step; determining a representative statistic of internal socket pressure during the gait cycle for a step, wherein the representative statistic includes one of an average, a median, a mode, or an root mean square; normalizing all or part of the real time monitored internal socket pressure against the determined representative statistic of the internal socket pressure to generate a normalized pressure data for a step; and qualifying each normalized pressure data against a threshold value to generate a step quality data for a step.

In another embodiment, a system for intelligent vacuum suspension of a prosthetic socket is provided, the system can include: a prosthetic socket; an electric vacuum pump for creating a sub-atmospheric pressure within a prosthetic socket; a processor operatively connected to the electric vacuum pump and operable to control the electric vacuum pump; a communication interface operatively connected to the processor and operable to provide a communication channel to the processor; a pressure sensor for sensing an internal pressure of the prosthetic socket, the pressure sensor operatively connected to the processor; a power source operatively connected to the system and operable to provide power to the system; and a non-transient computer readable medium containing program instructions for causing the processor to perform the method of detecting changes in prosthetic suspension stiffness.

In another embodiment, a method for determining fit of a prosthetic socket is provided, the method can include: using a pressure sensor to collect internal socket vacuum suspension level data; processing the collected internal socket vacuum suspension level data to determine each of: (1) a current vacuum suspension level; and (2) internal socket motion; comparing the current vacuum suspension level against a vacuum suspension level threshold for a given activity by a user; comparing the internal socket motion against a socket motion threshold value; and communicating an adverse condition when a threshold value is exceeded.

In another embodiment, a method for identifying one or more transitions in vacuum suspension stiffness at one or more specific temporal locations in a gait cycle for a vacuum suspended prosthetic socket is provided, the method can include the steps of: detecting one or more local peaks for a relative motion between a lower limb prosthetic socket and a residual lower limb during the one or more specific temporal locations in the gait cycle.

In another embodiment, a method for intelligent vacuum suspension of a prosthetic socket is provided, the method can include the steps of: determining at least one vacuum suspension range for one or more activity levels; determining at least one residual limb motion threshold; real time monitoring of a vacuum suspension level; and real time monitoring of residual limb motion.

Any of the systems and methods shown and described herein can include the steps of: programming a processor of an intelligent vacuum suspension system with the determined at least one activity level threshold between the two or more activity levels, determined at least one vacuum suspension level range for the one or more activity levels, and the determined at least one residual limb motion threshold.

Any of the systems and methods shown and described herein can include the steps of: recording or sampling data associated with the real time monitored motion level; recording or sampling data associated with the real time monitored vacuum suspension level; and recording or sampling data associated with the real time monitored residual limb motion. Alternatively or additionally, any of the systems and methods shown and described herein can include the steps of storing at least one of: the recorded or sampled data associated with the real time monitored motion level; the recorded or sampled data associated with the real time monitored vacuum suspension level; and the recorded or sampled data associated with the real time monitored residual limb motion, for later evaluation by a user or practitioner.

Any of the systems and methods shown and described herein can include the step of triggering an adaptive vacuum response when a value of the real time monitored motion level exceeds the determined at least one activity level threshold between the two or more activity levels.

Any of the systems and methods shown and described herein can include the step of triggering an adaptive vacuum response when a value of the real time monitored vacuum suspension level exceeds the determined at least one vacuum suspension level range for the one or more activity levels.

Any of the systems and methods shown and described herein can include the step of triggering a relative motion vacuum compensation response when a value of the real time monitored residual limb motion exceeds the determined at least one residual limb motion threshold.

Any of the systems and methods shown and described herein can include the step of displaying data associated with at least one of: the real time monitored motion level; the real time monitored vacuum suspension level; and the real time monitored residual limb motion.

Any of the systems and methods shown and described herein can include qualifying and quantifying one or more steps taken by a prosthetic leg device using an intelligent vacuum suspension system during a swing phase of the gait cycle. The swing phase can include an early swing phase, a mid-swing phase, and a late swing phase.

Any of the systems and methods shown and described herein can include monitoring a vacuum waveform of the internal socket pressure for peaks in at least one of: an early portion, a middle portion, or a late portion of the vacuum waveform. The vacuum waveform can include a waveform including an early peak and a late peak. A maximum pressure of the late peak can be normalized against the determined average internal pressure to generate the normalized pressure data.

Any of the systems and methods shown and described herein can include monitoring a vacuum waveform of the internal socket pressure. The vacuum waveform can include an augmented middle peak, and a pressure around a midway point of the augmented middle peak. The pressure around the midway point of the augmented middle peal can be normalized against the determined average internal pressure to generate the normalized pressure data.

According to any of the systems and methods shown and described herein, an ideal normalized pressure data can be about 1. Alternatively or additionally, the threshold value is ± 1.5% of the ideal normalized pressure data.

Any of the systems and methods shown and described herein can display in a graphical format at least one of: a pressure waveform of the monitored internal socket pressure, the determined average internal socket pressure during the swing phase of the gait cycle, and the step quality data.

Any of the systems and methods shown and described herein can include the steps of sending the real time monitored internal socket pressure, the determined average internal socket pressure during the swing phase of the gait cycle, and the step quality data to a remote processor.

Any of the systems and methods shown and described herein can include an inertial sensing package. Alternatively or additionally, the inertial sensing package can include at least one accelerometer sensor. Alternatively or additionally, the inertial sensing package can include three accelerometer sensors. A first accelerometer sensor can be operable to measure acceleration in a x-direction relative to the prosthetic socket. A second accelerometer sensor can be operable to measure acceleration in a y-direction relative to the prosthetic socket. A third accelerometer sensor can be operable to measure acceleration in a z-direction relative to the prosthetic socket. Alternatively or additionally, the inertial sensing package can include at least one gyroscopic sensor. Alternatively or additionally, the inertial sensing package can include three gyroscopic sensors. A first gyroscopic sensor can be operable to measure rotation about a x-direction relative to the prosthetic socket. A second gyroscopic sensor can be operable to measure rotation about a y-direction relative to the prosthetic socket. A third gyroscopic sensor can be operable to measure rotation about a z-direction relative to the prosthetic socket.

According to any of the systems and methods shown and described herein, the communication interface can be a wireless communication interface operable to provide wireless communication to and from the processor.

Any of the systems and methods shown and described herein can include a fob. The fob can be operable to provide wireless communication to the communication interface and receive wireless communication from the communication interface.

According to any of the systems and methods shown and described herein, the processor can include a memory. The memory can be operable to store one or more programs for execution by the processor and store internal socket pressure data from the pressure sensor.

Any of the systems and methods shown and described herein can include a graphical user interface operable to provide instructions to the processor.

Any of the systems and methods shown and described herein can include a display for displaying a graphical user interface for providing instructions to the processor and displaying data sent from the processor.

According to any of the systems and methods shown and described herein, the adverse condition can be improper socket volume relative to a residual limb volume when the vacuum suspension level threshold is exceeded.

According to any of the systems and methods shown and described herein, the adverse condition adverse condition is improper socket shape relative to a residual limb shape when the socket motion threshold is exceeded.

According to any of the systems and methods shown and described herein, relative motion can be detected with a position sensor. Alternatively or additionally, the position sensor can be an inductive sensor. Alternatively or additionally, the position sensor can be a Hall sensor.

According to any of the systems and methods shown and described herein, relative motion can be detected by processing sampled data from a pressure sensor measuring an internal socket pressure. Alternatively or additionally, the sampled data can be processed from the pressure sensor measuring the internal socket pressure to evaluate one or more significant pressure deviations above an average pressure level during a swing phase of the gait cycle. Alternatively or additionally, the one or more significant pressure deviations can be evaluated by normalizing the one or more significant pressure deviations by the average pressure level during the swing phase of the gait cycle. Alternatively or additionally, the one or more significant pressure deviations can be evaluated by comparing the one or more significant pressure deviations to a threshold value. Alternatively or additionally, the sampled data can be processed from the pressure sensor measuring the internal socket pressure to evaluate significant pressure deviations from an average vacuum level during a stance phase of the gait cycle. Alternatively or additionally, the significant pressure deviations can be evaluated by normalizing the significant pressure deviations by the average vacuum level during the stance phase of the gait cycle and comparing a resultant normalized value to a threshold. Alternatively or additionally, the significant pressure deviations can be evaluated by comparing the significant pressure deviations to a threshold value.

According to any of the systems and methods shown and described herein, the residual limb motion can be monitored using a motion sensor. The motion sensor can include at least one of: a hall sensor, an inductive sensor, an ultrasonic sensor, and a force sensor.

According to any of the systems and methods shown and described herein, the residual limb motion can be monitored by processing pressure data from a pressure sensor in communication with an air space between a residual limb and a socket.

According to any of the systems and methods shown and described herein, pressure data can be processed by comparing an average of sampled data from one portion of a gait cycle to: one or more average values, one or more minimum values, or one or more peak values for one or more subsets of the averaged sampled data. Alternatively or additionally, vacuum suspension can be monitored during a swing phase of a gait cycle. Alternatively or additionally, the values of the gait cycle that are compared to a mean suspension level can be from at least one of: an early swing phase peak, a middle swing phase peak, or a late swing phase peak. Alternatively or additionally, the pressure data can be processed by comparing a root meet square (RMS) of sampled data from one portion of a gait cycle to one or more: RMS values, average values, minimum values, or peak values for one or more subsets of the root mean squared sampled data. Alternatively or additionally, the vacuum suspension can be monitored during a stance phase of a gait cycle. Alternatively or additionally, vacuum suspension can be monitored during a heal strike transition or a toe off transition of a gait cycle.

Any of the systems and methods shown and described herein can include the step of programming a processor of an intelligent vacuum suspension system with the determined at least one vacuum suspension level range for the one or more activity levels, and the determined at least one residual limb motion threshold.

Any of the systems and methods shown and described herein can include the steps of: recording or sampling data associated with the real time monitored vacuum suspension level; and recording or sampling data associated with the real time monitored residual limb motion.

Alternatively or additionally, the systems and methods shown and described herein can include the step of storing at least one of: the recorded or sampled data associated with the real time monitored vacuum suspension level; and the recorded or sampled data associated with the real time monitored residual limb motion, for later evaluation by a user or practitioner.

Any of the systems and methods shown and described herein can include the step of triggering a relative motion vacuum compensation response when a value of the real time monitored residual limb motion crosses the determined at least one residual limb motion threshold.

Any of the systems and methods shown and described herein can include the step of displaying data associated with at least one of: a real time monitored motion level; the real time monitored vacuum suspension level; and the real time monitored residual limb motion.

According to any of the systems and methods shown and described herein, the relationship between pressure variation of the internal pressure of the prosthetic socket and the relative socket motion of the prosthetic socket can be for a single patient at a single activity level.

According to any of the systems and methods shown and described herein, the one or more sensors comprise a pressure sensor. The data indicative of the relative socket motion of the prosthetic socket can be a pressure waveform provided by the pressure sensor. The processor can execute the program instructions to automatically: detect one or more peaks in the pressure waveform; and quantify the relative socket motion based upon the one or more peaks detected in the pressure waveform. Alternatively or additionally, an inertial sensing package can monitor motion of the prosthetic socket. The processor can execute the program instructions to automatically identify a gait cycle for a step from the motion of the prosthetic. The gait cycle can include phases. The one or more peaks can be correlated to each of the phases of the gait cycle. Alternatively or additionally, the at least one phase can include an early swing phase, a mid-swing phase, and a late swing phase. Alternatively or additionally, the processor can execute the program instructions to automatically normalize a magnitude of the one or more peaks of each of the phases into normalized values for each of the phases. The normalized values for each of the phases can be combined into a sum. Alternatively or additionally, the processor can execute the program instructions to automatically qualify the vector sum of the step against a threshold value. A step quality value can be generated for the step. Alternatively or additionally, a communication interface can be in communication with the processor. The processor can execute the program instructions to automatically transmit the step quality value, the vector sum, the normalized values, the pressure waveform, or a combination thereof via the communication interface. Alternatively or additionally, the processor can execute the program instructions to automatically normalize the one or more peaks into a normalized value. The normalized value of the one or more peaks can be compared with a socket motion threshold value. Alternatively or additionally, the one or more peaks can be normalized into the normalized value using a pressure statistic of at least a portion of the pressure waveform. The pressure statistic can include an average, a median, a mode, a root mean square, or a combination thereof. Alternatively or additionally, the processor can execute the program instructions to automatically communicate an adverse condition when the socket motion threshold value is exceeded.

According to any of the systems and methods shown and described herein, an inertial sensing package can monitor motion of the prosthetic socket. The processor can execute the program instructions to automatically determine an activity level based upon the motion of the prosthetic socket. The vacuum suspension range can be determined based in part upon the activity level. Alternatively or additionally, the inertial sensing package can include at least one accelerometer sensor, at least one gyroscopic sensor, at least one force sensor, at least one pressure sensor, or a combination thereof.

According to any of the systems and methods shown and described herein, the one or more sensors can include a motion sensor that directly senses the relative socket motion of the prosthetic socket. Alternatively or additionally, the motion sensor can include at least one of a hall sensor, an inductive sensor, an ultrasonic sensor, and a force sensor.

According to any of the systems and methods shown and described herein, a step can be identified via the motion data of the prosthetic socket. The step can be correlated to the data indicative of relative socket motion of the prosthetic socket. The data indicative of relative socket motion of the prosthetic socket that is correlated to the step can be normalized into normalized data. The step can be classified according to the normalized data.

According to any of the systems and methods shown and described herein, the data indicative of the internal pressure of the prosthetic socket and the data indicative of the relative socket motion of the prosthetic socket can be compared to expected level of vacuum data. The expected level of vacuum data can correspond to the activity level associated with the data indicative of the internal pressure of the prosthetic socket and the data indicative of the relative socket motion of the prosthetic socket. The expected level of vacuum data can include pressure data and relative socket motion data corresponding to a proper fit. A fit of the prosthetic socket can be evaluated based upon a mismatch of the data indicative of the internal pressure of the prosthetic socket and the data indicative of the relative socket motion of the prosthetic socket compared to the expected level of vacuum data.

According to any of the systems and methods shown and described herein, a graphical user interface can be provided upon a display operatively connected to a remote processor driven device. The remote processor driven device can be in communication with the processor. The graphical user interface can present at least one of the motion data of the prosthetic socket, the data indicative of the internal pressure of the prosthetic socket and the data indicative of the relative socket motion of the prosthetic socket. Suspension level input can be received via the graphical user interface. One or more of the multiple suspension levels can be changed based upon the suspension level input.

According to any of the systems and methods shown and described herein, the processor can execute the program instructions to automatically select a vacuum suspension range. The selection can be at least partially informed by the quality of the suspension. The processor can execute the program instructions to automatically actuate the electric vacuum pump. The internal pressure of the prosthetic socket can be adjusted to be within the vacuum suspension range.

Any of the systems and methods shown and described herein can include a display operatively connected to a remote processor driven device. The remote processor driven device can be in communication with the processor. The remote processor driven device can automatically receive the quality of the suspension from the processor. The remote processor driven device can automatically provide a graphical user interface upon the display. The graphical user interface can be configured to evaluate or tune operation of the electric vacuum pump.

According to any of the systems and methods shown and described herein, the processor can execute the program instructions to automatically store the quality of the suspension to the non-transient computer readable medium.

## BRIEF DESCRIPTION OF THE DRAWINGS

In the accompanying drawings, structures are illustrated that, together with the detailed description provided below, describe example embodiments of the claimed invention. Where appropriate, like elements are identified with the same or similar reference numerals. Elements shown as a single component may be replaced with multiple components. Elements shown as multiple components may be replaced with a single component. The drawings may not be to scale. The proportion of certain elements may be exaggerated for the purpose of illustration.

**FIG. 1** illustrates an example prosthetic socket with electric vacuum pump.

**FIG. 2** illustrates an example schematic diagram of a processor controlled intelligent vacuum suspension system.

**FIG. 3** illustrates an example graphical user interface for intelligent vacuum suspension system.

**FIG. 4** illustrates an example graphical user interface for intelligent vacuum suspension system.

**FIG. 5** illustrates an example graphical user interface for intelligent vacuum suspension system.

**FIG. 6** illustrates an example graphical user interface for intelligent vacuum suspension system.

**FIG. 7** illustrates an example graphical output showing internal socket pressure variations for different levels of vacuum suspension.

**FIG. 8A** illustrates example socket motion relative to a user's residual limb.

**FIG. 8B** illustrates example socket motion relative to a user's residual limb.

**FIG. 8C** illustrates example socket motion relative to a user's residual limb.

**FIG. 9** illustrates a graph showing an example effect of vacuum level on pressure/motion profile.

**FIG. 10** illustrates sample pressure waveforms for characterizing socket motion type.

**FIG. 11** illustrates a flowchart of an example method for adaptive vacuum control.

**FIG. 12A** illustrates a graphical representation of step classification.

**FIG. 12B** illustrates a graphical representation of step classification.

**FIG. 13** illustrates an example graphical user interface for step classification.

**FIG. 14** illustrates a flowchart of an example method for step classification.

**FIG. 15** illustrates a flowchart of an example method for determining proper socket fit.

**FIG. 16** illustrates a flowchart of an example method for tracking a patient's socket fit.

## DETAILED DESCRIPTION

Adaptive vacuum suspension using an intelligent vacuum suspension control improves upon existing adaptive vacuum suspension control by providing enhanced adaptive vacuum control and providing vacuum suspension feedback diagnostics to a practitioner/prosthetist.

Referring again to **FIG. 1,** intelligent vacuum suspension control may be incorporated into existing vacuum suspension systems **100** using socket **102** and electric vacuum pump **104a** and **104b.** Vacuum pump **104a** may be in line with socket **102** and prosthetic limb (not shown). Alternatively, vacuum pump **104b** may be mounted on a side of socket **102.** Further, the pump might be mounted on another location of the prosthesis such as the pylon or foot. Both vacuum pump **104a** and **104b** provide evacuation of air within socket **102** to create a negative pressure (vacuum) and suspend socket **102** from a user's residual limb. A vacuum pump for evacuating air from socket **102** need not be limited to embodiments illustrated by vacuum pump **104a** and **104b** and a vacuum pump may be of any design or location on, in, or remote from socket **102.**

Referring to **FIG. 2,** a schematic of an example processor **206** controlled intelligent vacuum suspension system **201** operatively connected to an example prosthetic device **100** is illustrated. Processor **206** may be integrated into a prosthetic device, such as prosthetic device **100.** Intelligent vacuum suspension system **201** may include a CPU, microprocessor, or other processor **206** operable to: be programmed to accept one or more signal inputs; process one or more signal inputs based on instructions stored in a memory of processor **206;** and provide one or more output signals based on processed input signals. Intelligent vacuum suspension system **201** may further include one or more sensors **208,** vacuum pump **204,** communication interface **210,** and power source **212,** all operatively connected to processor **206.** Sensors **208** may include any or all of gyroscopic sensors, accelerometers, induction sensors, magnetometers, hall sensors, optical sensors, pressure sensors, temperature sensors, and like sensors. Sensors **208** may be operable to take one or more data readings of one or more conditions (i.e. pressure, acceleration, velocity, displacement, orientation, momentum, magnetic field, inductance, etc.), both discretely and continuously, to provide one or more input signals to processor **206.** Vacuum pump **204** may be actuated and controlled by processor **206** in response to an input from sensors **208,** based on programming of processor **206,** in response to other signals received and processed by processor **206** (i.e. a remote signal from a fob, cell phone, and the like), and by other conditions not controlled by processor **206** (i.e. a user's manual actuation of vacuum pump **204**). Communication interface **210** may be used to communicate with processor **206** to provide further input/output to processor **206** and act as a communication channel to/from processor **206.** Communication interface **210** may be a hardwired connection such as: a USB connection connected via a USB cable, or serial connection connected via a serial cable, or an Internet connection using an Ethernet cable; a transceiver capable of wirelessly transmitting and receiving signals to and from processor **206** which may include use of WiFi and other 802.11 standards, Bluetooth ®, cellular communications, and the like; an input/output pin or other electrical connection to processor **206** capable of inputting or outputting a signal to and from processor **206;** a slot or drive capable of processing a memory device or computer readable media such as an optical medium (i.e. compact disc, Bluray, and the like), magnetic media (floppy disk, hard disk, and the like), solid-state drive, memory card, and the like. Additionally, communication interface **210** may further process data so as to encrypt data prior to communicating data from processor **206** to another device. Communication interface **210** may also communicate data as a text message (SMS), audio signal, video signal, or any combination thereof. In one embodiment, communication interface **210** may include more than one interface such as a main communication interface **210** and intermediary communication interfaces **210.** Power source **212** may be any known power source used to power processor **206** such as one or more batteries, capacitors, a hard wired power connection, or a generator. Processor **206** may include additional memory for storing instructions, accumulated data, and the like. Processor **206** is capable of reading and executing any non-transient, computer readable medium containing program instructions for performing any of the methods disclosed herein, such that system **201** is operable to perform the methods as disclosed herein.

One or more graphical user interfaces (GUIs) **314** may be used to provide initial parameters and initialize processor **206** for intelligent vacuum suspension system **201.** GUIs **314** may be implemented on any remote processor driven device with a display such as a computer, smart phone, tablet, remote control, fob, and the like. In one embodiment, GUI **314** is only available to a practitioner for customizing and programming vacuum suspension parameters, checking diagnostics and adverse condition alerts, and communicating to and from processor **206** of intelligent vacuum suspension system **201.** In another embodiment, a user can access GUIs **314** to provide input parameters to, and check diagnostic messages from intelligent vacuum suspension system **201.** In yet another embodiment, remote access by the practitioner or manufacturer could be used to assist or train the user or service the suspension system. Understandably, hardware from the aforementioned processor driven devices and software of GUI **314** may send input parameters entered into GUI **314** to communication interface **210** which transfers input parameters to processor **206** for control of vacuum pump **204.** Similarly, data output from processor **206** via communication interface **210** may be received by hardware on a remote processor driven device, and software of GUI **314** may process output data from processor **206** for display on GUI **314.**

Referring to **FIGS. 3** and **4,** example GUIs **314** are illustrated. In **FIG.** 3, an example initialization GUI **314** is provided for a practitioner to enter input parameters. Suspension level (set point/upper set point/USP) **318** may be set, and a suspension range **320** may be provided for suspension level **318.** Suspension range **320** may be employed to provide a functional suspension level for an activity while conserving system power, and minimizing the number of activations of the pump which can result in excessive distractions for the user. For example, a practitioner and user may indicate that a vacuum level of 12 inHg to 18 inHg may provide sufficient suspension for an activity such as walking. Range **320** for this activity may be communicated to processor **206** and vacuum pump **204** may remove air from within socket **102** until a vacuum suspension level of 18 inHg, the upper set point, or set point **318** is achieved. In a typical socket **102,** the vacuum suspension level within socket **102** will continually and slowly decrease during use of prosthetic device **100,** typically due to leaks through imperfections in one or more seals of socket **102.** In an effort to save power and prevent vacuum pump **204** from continually trying to maintain a vacuum suspension level of 18 inHg within socket **102,** range **320** may be provided to allow for a reduction in vacuum suspension level to a lower set point **319** of 12 inHg before actuating vacuum pump **204** to restore suspension levels to upper set point **318.** Practitioner and/or user may agree through a course of testing vacuum suspension limits during a consultation that range **320** provides an adequate suspension level for a given activity and is comfortable for a user. A maximum set point **322** may be a practitioner and/or user defined maximum value that provides suspension while still being comfortable for a user. Suspension levels past maximum set point **322** may cause discomfort to a user and negatively affect circulation within the user's residual limb. Suspension levels below lower set point **319** may allow cyclical or other undesirable movement of socket **102** relative to a user's residual limb. These lower vacuum levels can correspond to a risk of excessive displacement of socket **102** relative to a user's residual limb. Such excessive displacement can compromise the interaction between a user's residual limb and the socket **102,** as such these lower vacuum levels cannot be maintained without risk of injury to a patient's limb. Battery indicator **324** may show a level of charge or power available to power source **212.**

As may be appreciated, a patient may move or walk or otherwise travel through their environment with a range of intensities, speeds, or cadences thus exposing a prosthetic device to different combinations of accelerations and/or rotations at these various types of activity. For the purpose of clarity, as used herein, a patient's given speed, cadence, pattern, or intensity of activity while using a prosthetic device may be known as a "level of activity" or an "activity level."

Referring to **FIG. 4,** GUI **314** illustrates an example adaptive vacuum GUI **426** for setting ranges **420a** and **420b** for two or more activity levels **428a** and **428b.** As described before, each activity level **428a** and **428b** may include its own vacuum suspension level set point **418a, 418b,** lower set point **419a, 419b,** and range **420a, 420b.** A user maximum set point **322** and charge indication **324** may also be provided. Indicator arrow **430** may output pressure data from sensor **208** to indicate a current vacuum suspension level within socket **102** in real time.

In addition, sensor **208** may also include an inertial sensing package to monitor motion of prosthetic socket **102,** and accordingly motion of a user. The inertial sensing package can comprise at least one accelerometer sensor, at least one gyroscopic sensor, at least one force sensor, at least one gyroscope, at least one pressure sensor, or a combination thereof. In some embodiments, the inertial sensing package can be configured to sense data along multiple dimensions, i.e., two dimensional data, three dimensional data or the like. In one embodiment, inertial sensing package **208** may include 3 accelerometers with a first accelerometer sensing acceleration data in a x direction relative to prosthetic device **100,** a second accelerometer sensing acceleration data in a y direction relative to prosthetic device **100** and a third accelerometer sensing acceleration data in a z direction relative to prosthetic device **100.** Data sensed by each accelerometer may be squared and added together to give a scalar value of a square of the magnitude. Intrinsic functions such as taking a square root of a number may be cumbersome for small microprocessors such as might be employed as processor **206.** Accordingly, a square of a magnitude may be used interchangeably with a magnitude since both will be monotonic increasing functions. Magnitude data from inertial sensing package **208** may be more easily processed and stored by processor **206** since use of a magnitude eliminates issues such as orientation of sensors **208** and/or prosthetic device **100.** Accordingly, data from inertial sensing package **208** may be used to sense different activity levels **428a** and **428b** with processor **206** and communication interface **210** processing and sending activity level data to a remote processor driven device for display on GUI **426.**

Different activity levels **428a** and **428b** may correspond to any of a variety of activity levels, including for example moving and resting, respectively. Possible activity levels that may be processed include, for example, sitting, resting, standing, walking, jogging, sprinting, jumping, and the like. Any number of activity levels **428a** and **428b** may be processed by processor **206.** GUI **426** may provide a graphical representation of activity level **428a** and **428b.**

Similarly, range **420a** or **420b** and set point **418a** or **418b** data may be entered into GUI **426** and communicated to processor **206,** so that when inertial sensing package **208** senses accelerations or rotations and transfers this data to processor **206,** processor **206** may then convert this motion information into a measure of activity and subsequently actuate electric vacuum pump **204** to obtain a desired vacuum suspension level **418a** or **418b** based on an activity level. Accordingly, providing processor **206** with vacuum ranges **420a, 420b** and set point **418a, 418b** data for two or more activity levels **428a** and **428b,** and one or more activity thresholds 536, provides a level of adaptive vacuum for intelligent vacuum suspension system **201.**

Alternatively or additionally, the motion of prosthetic socket **102** detected by the inertial sensing package **208** can be utilized to identify a gait cycle for each step taken by the user. Specifically, the gait cycle can comprise two major phases - a stance phase and a swing phase. The stance phase can correspond to the portion of the gait cycle where the prosthetic device **100** contacts a walking surface. The stance phase can span from a heel strike, where the heel of the prosthetic device **100** first contacts the walking surface during the step, to a toe off, where the toe of the prosthetic device **100** last contacts the walking surface during the step. The stance phase can furthermore comprise a single support phase. During the single support phase, the weight of the user can be substantially completely supported by the prosthetic device **100,** i.e., the other leg can be out of contact with the walking surface. The swing phase can span from the toe off to a heel strike of the subsequent step. During the swing phase the prosthetic device **100** can be out of contact with the walking surface. In some embodiments, the gait cycle can be divided at specific temporal locations. For example, the swing phase can start after the toe off event and be comprised of an early swing phase, a mid-swing phase, and a late swing phase, and then end with the heel strike event. Accordingly, the gait cycle can be correlated with other data collected by the prosthetic device **100** via particular phases, temporal locations, and the like.

Referring to **FIG. 5,** an example GUI **314** illustrating an activity threshold and activity capture GUI **532** is provided. Activity threshold GUI **532** may be used by a practitioner or user to monitor activity data **534** for different activity levels **428a** and **428b** and set an activity threshold **536.** Activity threshold **536** may be used by processor **206** for adaptive vacuum control. If inertial sensing package **208** senses an activity level above or below this threshold, processor **206** may actuate electric vacuum pump **204** to adjust a vacuum level within socket **102** to a proper suspension level **418a, 418b** based on sensed activity level. While two activity levels **428a** and **428b** are illustrated for exemplary purposes, intelligent vacuum suspension control may be modified to include numerous activity levels. GUI **532** may use a slider bar **538** to set activity threshold **536** or simply allow a graphical level bar on the plot of GUI **532** to be grabbed and moved with a cursor. In addition to the these methods allowing the practitioner or user to select an activity threshold, the GUI can also suggest or select a threshold based on calculations utilizing the activity data 428a and 428b.

Referring to **FIG. 6,** GUI **314** illustrating an example data analysis and diagnostic GUI **640** is illustrated. A practitioner or user may be able to select a date range at **642** to limit data to a specific time frame. Other status indicators such as charging **644,** leaks **646,** battery **648,** seal quality **650,** and relative limb motion **652** may be used with normal operation indicated by a check **654** for each. GUI **640** may allow for more in depth analysis of each category, for example, by clicking a mouse, keyboard, or other input device on a specific category if a problem has been indicated. An indication of problems may be shown by symbolic representation such as an exclamation point (!) **656,** different color text, or dialog box **657.**

## Socket Motion Sensing and Relative Motion Compensation

One primary cause of injury to a residual limb may be excessive motion of a residual limb within and relative to the prosthetic socket. In addition to jeopardizing tissue health, socket motion may be a serious adverse condition that may affect suspension of socket **102** from a user's residual limb, and impair a user's proprioception such that the user is less aware of the spatial orientation and movement of a prosthetic device. A properly evacuated socket, that is a socket with proper vacuum suspension level, may help to maintain both residual limb volume over time, and proper contact between the residual limb and the socket by creating an appropriate level of suspension force and thereby greatly reducing a tendency of a residual limb to move within a prosthetic socket. However, because a user's limb volume may vary over the course of a day, and/or fluctuate based upon weight, food intake, and the like; and because a patient's level of activity may at times fall outside of or between the expected ranges of activity, it may be difficult to know what level of vacuum may actually be necessary for a given user, at a given activity level, on a specific day. Predefined set points **418a/418b, 419a/419b,** and **420a/420b** for various activity levels **428a, 428b** may not adequately address adverse conditions arising from change in volume to a user's residual limb. As such, additional information about, and greater control of adaptive vacuum suspension levels may be necessary to provide more appropriate prosthetic suspension. To help facilitate such an improvement in suspension, sensor **208** within socket **102** may be used to sense residual limb motion within socket **102** and enable processor **206** to adjust a vacuum level therein to correct or compensate for socket motion. This additional level of vacuum compensation may be referred to as relative motion compensation or socket motion compensation. Over time, a map of activity level vs. vacuum level may be constructed and referenced to allow for quicker vacuum adjustments during either real time adaptive vacuum control, or diagnostic use by a practitioner.

As may be appreciated, relative motion, slop, or lash between the patient's residual limb and the prosthetic socket is generally undesirable. Because there are a number of moving components and systems associated with a patient wearing a prosthetic socket moving through their environment, in this document, for clarity, this type of relative motion between a patient's residual limb and their prosthetic socket will be referred to as "socket motion," "relative socket motion," or "residual limb motion." As may also be appreciated, a patient may move or walk or otherwise travel through their environment with a range of intensities, speeds, or cadences thus exposing their prosthesis to different combinations of accelerations and/or rotations at these various types of activity. Again, as used herein, a patient's given speed, cadence, pattern, or intensity of activity while using a prosthetic device may be known as a "level of activity" or an "activity level."

Several types of sensors **208** may be used to sense motion of a residual limb within socket **102.** For example, a motion sensor **208** may be comprised of a Hall sensor placed in a base of a prosthetic socket and a small magnet fastened to a tip of a prosthetic liner worn over a user's residual limb. Alternatively, a motion sensor **208** may be comprised of a mutual inductance device that measures a mutual inductance between a coil in a base of a prosthetic socket, and a small coil or conductive target placed on a tip of a prosthetic liner worn over a user's residual limb. A metallic or otherwise conductive thread sewn into an end of a residual limb liner in conjunction with an inductance sensor **208** in socket **102** of a prosthetic device **100** may also be used to sense motion of a residual limb relative to prosthetic socket **102.** In one embodiment, a motion sensor **208** is comprised of an ultrasonic sensor **208** that may be tuned to detect a target mounted on a tip of a prosthetic liner worn over a user's residual limb where said target is chosen to have an appropriate acoustical impedance to efficiently reflect a relevant acoustical signal. Placing ultrasonic sensor **208** in a prosthetic socket **102** may also directly detect a residual limb or prosthetic liner. In another embodiment, a motion sensor **208** includes a force sensor **208** placed in a bottom of a prosthetic socket **102.** Intermittent contact of a user's residual limb with a force sensor **208** may indicate an occurrence of residual limb motion within socket **102.** Use of aforementioned sensors **208** may indicate motion of residual limb within socket **208,** but data from a single inductive sensor **208** may or may not allow for characterization of residual limb motion relative to socket **102.** Such characterization may require multiple sensors depending on placement.

A pressure sensor **208** may also be used to sense motion of a user's residual limb relative to socket **102,** characterize the type of motion, and provide feedback data for either real time adaptive vacuum control, or diagnostic use by a practitioner.

Because socket **102** may be thought of as a sealed vessel, the Ideal Gas Law may be used to approximate internal socket pressure and socket motion relative to a user's residual limb where:

$$PV = nRT \quad \text{(Equation 1)}$$

where *P* is an internal socket pressure of socket **102,** *V* is a volume of air between socket **102** and a patient's limb or liner, *n* is an amount of air in socket **102,** *R* is the ideal gas constant, and *T* is a temperature of air within socket **102.** Both *n* and *R* may be assumed to be fixed values and *T* may be unlikely to change significantly after socket **102** has been worn for a period of time such as, for example, several minutes or about 10 minutes. This assumption of isothermal performance is a reasonable approximation of an actual system. However, an actual performance may be some place between isothermal and isentropic, such that scaling of Equation 1 would occur, but it would not affect the overall conclusion. Thus, socket **102** may be modeled as a closed system and Equation 1 may be simplified to Boyle's law where:

$$P \propto \frac{1}{V} \quad \text{(Equation 2)}$$

where *P*, internal socket pressure of socket 102, is inversely proportional to *V,* a volume of air between socket 102 and a liner or residual limb. Use of this relation may be used to sense motion of a user's residual limb within socket **102.** Further, pressure data and graphical representations of pressure data may be used to characterize socket motion both for purposes of adaptive vacuum suspension level control and to provide a practitioner with a visual feedback for detecting, characterizing, and correcting socket motion.

Referring to **FIG.** 7, graph **758** illustrates pressure waveforms for variations in vacuum pressure while walking at several different vacuum suspension levels. Waveform **760** showing vacuum pressure level variations within socket **102** at a suspension level of 18 In Hg shows a relatively symmetrical waveform similar to a sine wave. Waveform **762** showing vacuum pressure level variations within socket **102** at a suspension level of 8 In Hg, similar to waveform **760,** shows a relatively symmetrical waveform similar to a sine wave, but with an increase in amplitude. Waveform **764** showing vacuum pressure level variations within socket **102** at a suspension level of 4 In Hg shows an irregular waveform with an increase in amplitude with early and late peaks in the waveform. Waveform **766** showing a vacuum pressure level variation within socket **102** with bare minimum suction, shows an irregular waveform with a higher amplitude than the others with early and late peaks. Accordingly, shapes of waveforms associated with vacuum pressure variation may be used to classify a type of socket motion relative to a user's residual limb. Pressure variation data associated with each waveform may trigger an adaptive vacuum response from processor **206.**

Referring to **FIGS. 8A, 8B,** and **8C,** example types of motion **868** and **874** between a user's residual limb **876** and socket **102** are illustrated. Different vacuum suspension levels may cause socket motion characterized by: (1) a linear motion **874** of a user's residual limb **876** relative to socket **102** which may be characterized as "pistoning" **874,** as this motion mimics a movement of a piston in a cylinder; and (2) a rotational motion **868** of user's residual limb **876** relative to socket **102** which may be characterized as "rocking" **868,** as this motion mimics a backwards and forward rocking motion.

Referring to **FIG. 9,** a graph showing an example effect of vacuum level on pressure/motion profile is illustrated. In **FIG. 9,** data points for variations in vacuum (pressure) are plotted against socket motion (distance) for a single patient at a single cadence. Accordingly, slopes of the two lines **980** and **982** illustrated in **FIG. 9** are given by pressure/distance which gives a corresponding stiffness value. Noticeably, the stiffness factor for high vacuum levels shows little motion for a corresponding change in vacuum level as represented by line **980.** This stiffness represented by a slope of line **980** is about fivefold that for lower vacuum suspension levels which permit increased socket motion for similar changes in socket pressure as represented by line **982.** Accordingly, a range of suspension levels which exhibits little to no socket motion, as illustrated by line **980,** is unique for each patient. Determining an appropriate range of vacuum pressures within this region of high stiffness for each user for a given cadence may improve adaptive vacuum control by providing processor **206** with a range that may limit socket motion. In this example, when socket motion is detected, a practitioner or processor **206** may use past stiffness data in selecting a suitable vacuum suspension level for the patient under the current conditions.

As is noted above, the two lines **980** and **982** can be formed by collecting data points for a user for a given cadence or activity level. For a specific activity level, variations in vacuum and socket motion can be observed at known vacuum suspension levels, which are represented in **FIG. 9** by circular regions surrounding data points. Specifically, the two lines **980** and **982** were generated by observing variations in vacuum and socket motion at vacuum suspension levels of 20 in Hg, 14 in Hg, 8 in Hg, and passive suction. The process can be repeated for a desired number of activity levels, which can be determined as described herein. Thus, the stiffness data can comprise one or more sets of data (e.g., lines) that each correspond to a different activity level. In some embodiments, the stiffness data can be stored in memory that can be accessed to control vacuum suspension level. It is noted that, while stiffness is depicted as lines **980** and **982,** the stiffness data can be stored in memory in any machine readable format such as, but not limited to, look-up tables, equations, curves, boundary conditions, logical rules, an inflection point and the like. Further, simply storing the minimal vacuum level necessary to provide high stiffness suspension at a given activity level is sufficient for such a record.

Referring collectively to **FIGS. 8A, 8B, 8C and 9,** the stiffness data can be collected while the socket **102** is properly fitted to the residual limb **876.** For example, while the user is in consultation with a practitioner. Accordingly, the stiffness data can be utilized to evaluate fit problems associated with changes in the geometry of the residual limb **876** and the socket **102.** For example, over time , the residual limb **876** can change in shape, volume or both to create a mismatch with the socket **102.** Alternatively or additionally, through normal use, the geometry of the socket **102** can change in shape, volume or both due to fatigue or acute damage. Since the residual limb **876** can be obscured by the socket **102,** such changes can be difficult to observe. This can be further complicated by the losses of sensation in a residual limb often associated with injury or vascular disease.

The stiffness data can correspond to a relationship between variations in vacuum and the socket motion. Without being bound to theory, it is believed that the stiffness can correspond to the interaction of the residual limb **876** and the socket **102.** Specifically, high stiffness can correspond to a relatively large amount of resistance to relative motion, corresponding to solid contact with the residual limb **876.** Likewise, low stiffness can correspond to a relatively small amount of resistance to relative motion due to less solid contact with, or even separation from, the residual limb **876.** Applicants have discovered that the stiffness can by represented line **980** (high stiffness) and line **982** (low stiffness), which provide a discontinuity in the stiffness that demarcates a high stiffness region and a low stiffness region. Specifically, line **980** has a relative large slope compared to line **982.** Lines **980** and **982** can form a "knee" or "bend" in the continuum of stiffness data.

Moreover, the regions of low stiffness data can be correlated to socket motion and can therefore be utilized to predict and mitigate undesired socket motion. In the high stiffness region represented by line **980,** a relatively large variation in vacuum can correspond to a relatively small amount of socket motion. Accordingly, the residual limb **876** can be providing a relatively large amount of resistance to deformation, which can be correlated with a low amount of socket motion. In the low stiffness region represented by line **982,** a relatively small variation in vacuum corresponds to a relatively large amount of socket motion. Thus, the residual limb **876** can be providing a relatively small amount of resistance to deformation, which can be correlated with a high amount of socket motion.

In some embodiments, the stiffness data can be utilized to determine a vacuum suspension range. For example, the lowest suspension level necessary to remain on the high stiffness can be determined for a given patient at one or more activity levels. The patient's activity level can then be compared to this table and an appropriate level of suspension can be selected to keep the patients suspension in the region of high stiffness. Should the current combination of vacuum suspension range and activity level correspond to a region of low stiffness represented by line **982,** a new vacuum suspension range that corresponds to a region of high stiffness represented by line **980** can be selected. Without being bound to theory, at a fixed level of activity, the forces in the swing phase can be considered as consistent from step to step. Accordingly, an assumption of consistent force or loading can be utilized to apply stiffness data. Specifically, the stiffness data can identify some minimum level of vacuum for the high stiffness region (e.g., an inflection point), which could be treated as a desired level of vacuum. Moreover, when activity level is tracked, the activity level can be utilized to determine an appropriate suspension level from the stiffness data. Alternatively or additionally, peaking can be detected and can be utilized as an indirect indication that the stiffness data has broken down. That is, peaking can be an independent artifact indicative of operation in a region of low stiffness. Accordingly, peaking can be utilized to determine an appropriate suspension level without knowledge of cadence or activity level. It is furthermore noted, that a shift in the minimum level of vacuum for the high stiffness region can be indicative of a change in fit between the residual limb **876** and the socket **102.**

Referring to **FIG. 10,** sample pressure waveforms indicative of certain socket motion types **868** and **874** are illustrated. In some embodiments, the pressure waveforms, or any portion thereof, can be correlated to the gait cycle, as described above, to analyze the pressure waveforms. For example, waveform **1084** shows a shape profile suggesting pistoning-type socket motion **874,** while waveform **1086** shows a shape profile suggesting rocking type socket motion **868.** Pistoning **874** may occur when a vacuum level of an interior of socket **102** is not great enough to adequately secure socket **102** of prosthetic device **100** to residual limb **876** and may be caused by forces such as gravity and radial force (i.e. centrifugal force, etc.) that may act to pull socket **102** from a user's residual limb **876** while prosthetic device is in use (i.e. during a swing phase of ambulation). Rocking **868** may occur at vacuum levels insufficient to maintain rigid suspension during acceleration and deceleration of a residual limb at a start and end of the swing phase of the gait cycle. While a vacuum level within socket **102** may be sufficient to overcome forces that may cause pistoning **874,** motional forces (i.e. tangential force, torque, etc.) may still act upon socket **102** to cause rocking **868.**

Early and late peaking occurring on waveform **1086** is indicative of relative motion between the residual limb and the socket due to an imbalance of forces on socket **102** during a beginning **870** and end **872** of the swing phase of the gait cycle. Data points from a collection of waveforms similar to **FIG. 7** taken at a range of vacuum levels and a single cadence or activity level may be further processed to provide an activity threshold value for adaptive vacuum control at that cadence or activity level. For wave **1086,** associated with socket rocking motion **868,** a maximum late peak value **1088** may be taken for each step and normalized against an average swing phase vacuum suspension level for each step. Determination of a "late peak" waveform may be conducted by comparing the maximum amplitude during some fraction of the later portion of the swing phase portion of waveform **1086** with the average amplitude of the swing phase portion of waveform **1086.** Where the peak amplitude of the late portion of the swing phase is higher than the average amplitude of the wave, a "late peak" waveform may be identified. In one embodiment, normalization of a maximum late peak value **1088** against average vacuum suspension level during the swing phase of the gait cycle for each step gives a value close to 1.00 (one) when there is little late peaking, and values greater than 1.00 (one) are indicative of a late peak in an associated step.

Center peaking characterized by an augmented center peak **1090** on waveform **1084** may be indicative of relative motion between a user's residual limb and socket **102** due to an imbalance of forces acting on socket **102** during a midpoint **878** of the swing phase of the gait cycle. For wave **1084** associated with socket pistoning motion **874,** finding a minimum value of some center portion of the swing phase will capture a value at point **1092** from some portion of the augmented center peak 1090 and therefore yield a value greater than the average amplitude of the swing phase of waveform 1084. While simply detecting the peak value of some middle portion of the swing phase is somewhat accurate, this improved method of detecting a center peak value has the advantage of not capturing portions of a late peak that might overlap the center portion of the swing phase. The elevated level at **1092** of augmented center peak **1090** may be taken for each step and normalized against an average swing phase vacuum suspension level for each step. Determination of a "center peak" waveform may be conducted by comparing the amplitude at about the central portion of a wave of waveform **1084** to the average amplitude of the swing phase of waveform **1084.** Where the amplitude of the wave at about the central portion (which can include the center of the wave, a region surrounding the center of the wave, or both) of the wave is greater than the average amplitude of the wave, a "center peak" waveform may be identified. In one embodiment, normalization of a central minimum value **1092** of augmented center peak **1090** against average vacuum suspension level during the swing phase of the gait cycle for each step gives a value close to 1.00 (one) and values greater than 1.00 (one) are indicative of a center peak in the associated step.

Peaks in locations other than the swing phase of a of gait, such as an early or late portion of the swing phase as discussed above, may be detected in a similar manner, normalized by an average motion or pressure in some other portion of the gait cycle, and may be useful in evaluating a patient's suspension. As such, similar measures could, in this way, be extracted from the stance phase or the heel strike or toe off transitions of gait.

Effects of peaks in multiple portions of the gait cycle may be combined by treating the peaks as a collection of mutually orthogonal events and calculating a total magnitude of a resulting vector in *n* space where *n* is the number of peaking locations considered. While this assumption of independence is not strictly true, motion in one phase of the gait cycle does have an effect on other portions of the gait cycle, this construct can allow many different types of motion in socket **102** to be summarized in a single normalized value. In this way, a single parameter can capture all motion in a socket and be used as a single input to a suspension controller. Use of a system using center and late peaking values ($n = 2$) has been found to be useful for many different socket geometries and gait conditions. Sample data from a collection of patients using this methodology is shown in Figures 12a and 12b.

Deviations of normalized data from 1.00 (one) may be compared to a preset threshold for socket motion (i.e. a socket motion threshold) and used to trigger a vacuum level response (i.e. relative motion compensation) when normalized data exceeds the preset socket motion threshold. This threshold may be patient dependent and may be affected by socket geometry, socket size, patient tissue type, patient cadence or activity, and other factors. In one embodiment, a socket motion threshold of $\pm$ 1.5% of a normalized value from the average swing phase vacuum level has been found to be useful. In this manner, detection of steps with relative socket motion exceeding a threshold may provide an instant, real time adaptive vacuum level response to socket motion. As there is significant variation in the physiological makeup of individual users, there is also likely to be a variation in the appropriate motion threshold for a given user. In the case of socket pressure being used to detect motion, the scale of the pressure deviations seen when monitoring a socket is dependent on a number of factors including user weight, user activity level, socket fit, socket geometry, user tissue type, prosthesis weight, and others. In addition to this variability, this type of motion detection system has been shown to be even more sensitive to motion than most users. As a result, motion thresholds are likely to vary with users. While these examples describe control based on motion derived from pressure data, similar methods and calculations can be used to evaluate socket motion quantified by other aforementioned motions sensors.

With reference to **FIG. 11,** a flowchart illustrating one embodiment of a method **1100** which combines adaptive vacuum suspension with relative motion compensation. In one embodiment, method **1100** may include the following steps. One or more activity thresholds that divide two or more activity levels can be determined (**1101**). An initial vacuum suspension level range can be determined for the one or more activity levels (**1103**). A residual limb motion threshold can be determined (**1105**). An activity level can be monitored in real time to compare the activity level to one or more activity levels of a user. The method **1100,** may include the step of monitoring an activity level in real time, comparing the activity level to one or more activity levels of a user, and adjusting a vacuum suspension level (**1107**). In some embodiments, the vacuum suspension level can be adjusted when a monitored activity level exceeds a determined activity threshold. Data related to a monitored activity level can be recorded. Accordingly, the adaptive vacuum (**1107**) can adjust the suspension level when the monitored activity level is outside of the range set by the determined activity threshold. For example, the determined activity threshold can comprise a high activity level and a low activity level that corresponds to the current suspension setting. When the monitored activity level falls outside of the range defined by the high and low activity levels, the vacuum suspension level can be adjusted to be within the range. Thus, the vacuum level can be increased when a user becomes more active and decreased when a user returns to a lower level of activity. The control method **1100** can further comprise monitoring a vacuum suspension level in real time and adjusting a vacuum suspension level if a monitored vacuum suspension level falls outside the current vacuum suspension level range and recording data related to a monitored vacuum suspension level (**1109**); and monitoring residual limb motion in real time and adjusting a vacuum suspension level when a monitored residual limb motion exceeds a determined residual limb motion threshold and recording data related to monitored residual limb motion (**1111**). Control method **1100** combines traditional fixed vacuum suspension control (**1109**) with adaptive vacuum (**1107**) and relative motion compensation (**1111**). While this layering of control methodologies has been found to be useful, implementation of relative motion compensation does not require the use of these other methods of control.

Step Classification

In addition to real time adjustments to a vacuum setting based on a detection of "steps with motion," steps may be evaluated based on variations in pressure data and socket motion associated therewith and this information may then be either used to adjust a level of vacuum in the socket, or simply logged according to a hierarchy such as "no motion," "motion," and "marginal." While this example allows for 3 discrete ranges of motion, the results of such an evaluation may be subdivided into any number of ranges, or might be logged by independent motions and not a combination of motions such as that generated by the sum of squares process discussed earlier. In contrast to sorting into ranges, actual deviations or motion values may also be saved. Logged step data may be later retrieved and evaluated by a practitioner during a consultation with a user or by the user. In one embodiment, logged steps may be periodically sent from processor **206** via communication interface **210** to a practitioner or user using hardware discussed above. In another embodiment, logged steps may be periodically sent from processor **206** via communication interface **210** to an intermediary storage such as hard drive or cloud storage to be accessed by a practitioner or user.

An evaluation of steps may also be sent to a user via communication interface **210** if a socket motion threshold is exceeded for a signification portion of steps taken by the user. An evaluation of steps may be sent as a message, an audio message, a visual display, and the like.

An evaluation of steps may also be displayed to a practitioner or user in real time for evaluating vacuum suspension levels for a user. Real time evaluation of steps may allow a practitioner to make real time adjustment to vacuum suspension levels and evaluate a fit of socket **102.**

Referring now to **FIGS. 12A** and **12B,** a graphical representation of example step quality and quantity is illustrated for a system where late peaking and center peaking were combined (*n* = 2) and evaluated for a number of patients. Step classification **1294** may provide various indicia **1296** corresponding to how each step is classified. For example, indicia **1296** may use different colors, shapes, fills, and the like to represent aforementioned step hierarchy and socket motion type. While boundary **1298** appears to be an ellipse due to different scales on the two axes, in this embodiment, this boundary is actually a circle. In this case, circle **1298** may show a socket motion threshold value for normalized socket motion data for steps where the number of motions monitored is two (*n* = 2). A similar collection of motions with n=3, such as early, middle, and late peaking of the swing phase, could be bounded by a sphere. Steps shown outside circle **1298** may indicate steps with significant or undesirable amounts of relative socket motion.

Referring now to **FIG. 13,** an example GUI **314** showing a step classification GUI **1300** is provided. Step classification GUI **1300** may provide a practitioner or user with graphical representations of pressure sensor data such as pressure wave form **1302,** motion **1304** and step classification **1306.**

With reference to **FIG. 14,** a flowchart illustrating one embodiment of a method **1400** for classifying one or more steps (in terms of both quality and quantity) using an intelligent vacuum suspension system during a gait cycle is provided. In one embodiment, method **1400** may include the steps of monitoring an internal socket pressure in real time during a swing phase of a user's gait cycle for each step taken **(1401);** collecting a series of monitored internal socket pressure readings during a swing phase of a user's gait cycle for each step taken (**1403**); calculating an average internal socket pressure during a swing phase of a user's gait cycle for each step taken **(1405);** normalizing one or more portions of all or part of collected pressure waveform data against a calculated average internal socket pressure during the swing phase to generate a normalized pressure value for each step taken during a particular portion of the gait cycle (**1407**); combining one or more measures of internal socket motion by calculating a vector sum (**1408**), qualifying each combined measure of socket motion against a threshold value to generate a step quality value for each step taken (**1409**); and collecting a step quality data for each step taken (**1411**). While evaluation of the swing phase of gait has been found to be particularly useful, similar practices can also be used to extract useful motion information from other portions of the gait cycle.

Socket Fit

As use of a motion or pressure sensor **208** in intelligent vacuum suspension control **200** may be used to detect and characterize socket motion of socket **102** relative to a user's residual limb **876**, this data may also be used to evaluate and track a fit of prosthetic socket **102**. A proper shape and size of prosthetic socket **102** may be two important factors in determining proper socket fit. In one embodiment, a socket **102** of proper size and shape will limit socket motion at relatively low levels of vacuum suspension. Accordingly, this low level of vacuum may be characterized at the time of fitting, or a predictive algorithm may be employed to predict the necessary level of suspension necessary for a particular user and socket **102.** The level of suspension necessary to limit socket motion can then be monitored and compared to this expected level of vacuum for indications of issues with socket fit. Socket **102** with a proper shape and improper size-that is, shaped correctly based on a user's residual limb **876,** but of an improper volume, will have a tendency to produce pressure data characterized as showing little to no internal socket motion, but requiring vacuum suspension levels higher than those necessary with a proper fit for a given activity level. This necessary level of vacuum might be described as uncomfortable or even painful by the user depending on the degree to which the size is mismatched. Additionally, socket **102** with proper size and an improper shape will typically exhibit internal socket pressure data that suggests a need for both higher than necessary vacuum suspension level while still exhibiting increased socket motion. In this scenario, socket **102** may accommodate a correct volume of a user's residual limb **876,** but is shaped improperly relative to a user's residual limb **876.** As stated above, vacuum suspension levels higher than necessary for a properly fitting socket **102,** as exhibited in both of these scenarios, may negatively affect a user's residual limb **876** potentially inducing injury if the fit is sufficiently incorrect. An ability to detect improper fit for socket **102** may be used to monitor socket fit and indicate to the user and/or practitioner that a fit of socket **102** may be inappropriate and should be evaluated to prevent any negative effects. This process may be used both acutely, during a fitting of socket **102** in the practitioner's office, or to monitor socket fit over long periods of usage by the patient.

With reference to **FIG. 15,** a flowchart illustrating one embodiment of a method **1500** to evaluate acute fit of socket **102** at a fitting is provided. Method **1500** may include the steps of collecting and processing pressure sensor data across a range of vacuum levels and cadences or activity levels during gait (**1501**). Evaluating an amount of motion detected during gait at each level of vacuum, and then comparing amount of motion detected to an amount of vacuum that would be expected to be necessary to stabilize a specific patient's prosthesis **100** (assuming that the prosthesis may be suspended at a comfortable or achievable level of vacuum) (**1503**).

With reference to **FIG. 16,** a flowchart illustrating one embodiment of a method **1600** to track a patient's socket fit over longer periods of use is provided. Method **1600** may include the steps of having a patient move about (i.e. test movement) in a well-fitting socket at both a range of vacuum suspension levels and a range of cadences or activity levels (**1601**). Collecting socket motion data during patient test movement (**1603**). Constructing a curve based on collected socket motion data of a level of vacuum necessary to stabilize a patient's prosthesis as a function of activity level or cadence (**1605**). Recording or storing the curve for use by processor **206** (**1607**). Comparing current performance (either the necessary level of suspension to stabilize socket motion at a given cadence or activity level, or the amount of motion at the previously determined level of suspension for a given cadence or activity level) of socket **102** to recorded / stored curve during use of prosthesis 1**0**0. (**1609**) And notifying a patient and/or practitioner that it may be appropriate to have the patient visit the practitioner for a formal evaluation of socket fit if vacuum levels necessary to stabilize socket **102** increase by more than some acceptable limit (**1611**).

As mentioned previously, analysis of vacuum waveforms may be used to evaluate motion of socket **102** during various portions of the gait cycle. This information may be used to assist in tuning functionalities of a patient's prosthesis **100** beyond proper vacuum level. For instance, early and late peaking may generally be caused by rotational acceleration and deceleration of prosthesis **100.** For an above knee amputee, this acceleration may be communicated to prosthesis **100** through a prosthetic knee. While it may be possible to compensate for socket motion indicated by early and late peaking by adjusting vacuum levels, this motion may also be addressed by reducing knee torque in a prosthetic knee during a start and end of the swing phase of the gait cycle. To this end, gait information determined by socket motion evaluations may be used to provide performance data to other prosthetic components (for example, a prosthetic knee or foot), and thus further improve the overall performance of a complete prosthetic system. Other devices that might interface to such a complete prosthetic system so as to provide benefits to the patient include prosthetic feet, ankles, and generators.

Unless specifically stated to the contrary, the numerical parameters set forth in the specification, including the attached claims, are approximations that may vary depending on the desired properties sought to be obtained according to the exemplary embodiments. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

Furthermore, while the systems, methods, and apparatuses have been illustrated by describing example embodiments, and while the example embodiments have been described and illustrated in considerable detail, it is not the intention of the applicants to restrict, or in any way limit, the scope of the appended claims to such detail. It is, of course, not possible to describe every conceivable combination of components or methodologies for purposes of describing the systems, methods, and apparatuses. With the benefit of this application, additional advantages and modifications will readily appear to those skilled in the art. Therefore, the invention, in its broader aspects, is not limited to the specific details and illustrative example and exemplary embodiments shown and described. Accordingly, departures may be made from such details without departing from the spirit or scope of the general inventive concept. Thus, this application is intended to embrace alterations, modifications, and variations that fall within the scope of the appended claims. The preceding description is not meant to limit the scope of the invention. Rather, the scope of the invention is to be determined by the appended claims and their equivalents.

As used in the specification and the claims, the singular forms "a," "an," and "the" include the plural. To the extent that the term "includes" or "including" is employed in the detailed description or the claims, it is intended to be inclusive in a manner similar to the term "comprising," as that term is interpreted when employed as a transitional word in a claim. Furthermore, to the extent that the term "or" is employed in the claims (e.g., A or B) it is intended to mean "A or B or both." When the applicants intend to indicate "only A or B, but not both," then the term "only A or B but not both" will be employed. Similarly, when the applicants intend to indicate "one and only one" of A, B, or C, the applicants will employ the phrase "one and only one." Thus, use of the term "or" herein is the inclusive, and not the exclusive use. See Bryan A. Garner, A Dictionary of Modern Legal Usage 624 (2d. Ed. 1995). Also, to the extent that the terms "in" or "into" are used in the specification or the claims, it is intended to additionally mean "on" or "onto." To the extent that the term "selectively" is used in the specification or the claims, it is intended to refer to a condition of a component wherein a user of the apparatus may activate or deactivate the feature or function of the component as is necessary or desired in use of the apparatus. To the extent that the term "operatively connected" is used in the specification or the claims, it is intended to mean that the identified components are connected in a way to perform a designated function. Finally, where the term "about" is used in conjunction with a number, it is intended to include ± 10% of the number. In other words, "about 10" may mean from 9 to 11.

STATEMENTS

1. A system for intelligent vacuum control, the system comprising:

a prosthetic socket;
an electric vacuum pump that creates a sub-atmospheric pressure within the prosthetic socket;
one or more sensors that sense an internal pressure of the prosthetic socket and data indicative of relative socket motion of the prosthetic socket;
a non-transient computer readable medium comprising program instructions and stiffness data, wherein the stiffness data corresponds to a relationship between pressure variation of the internal pressure of the prosthetic socket and the relative socket motion of the prosthetic socket; and
a processor operatively connected to the electric vacuum pump, the one or more sensors, and the non-transient computer readable medium, wherein the processor executes the program instructions to automatically:

compare the data indicative of the relative socket motion of the prosthetic socket and the stiffness data;
determine a vacuum suspension range based upon the comparison of the data indicative of the relative socket motion of the prosthetic socket and the stiffness data; and
actuate the electric vacuum pump, whereby the internal pressure of the prosthetic socket is adjusted to be within the vacuum suspension range.

2. The system of statement 1, wherein:

the relationship of the stiffness data comprises a first slope of the pressure variation of the internal pressure of the prosthetic socket over the relative socket motion of the prosthetic socket;
the relationship of the stiffness data comprises a second slope of the pressure variation of the internal pressure of the prosthetic socket over the relative socket motion of the prosthetic socket;
the first slope is greater than the second slope; and
the vacuum suspension range corresponds to the first slope of the relationship of the stiffness data.

3. The system of statement 1, wherein the relationship between pressure variation of the internal pressure of the prosthetic socket and the relative socket motion of the prosthetic socket is for a single patient at a single activity level.

4. The system of statement 1, wherein:

the one or more sensors comprise a pressure sensor;
the data indicative of the relative socket motion of the prosthetic socket is a pressure waveform provided by the pressure sensor; and
the processor executes the program instructions to automatically:

detect one or more peaks in the pressure waveform; and
quantify the relative socket motion based upon the one or more peaks detected in the pressure waveform.

5. The system of statement 4, comprising an inertial sensing package that monitors motion of the prosthetic socket, wherein the processor executes the program instructions to automatically:

identify a gait cycle for a step from the motion of the prosthetic, wherein the gait cycle comprises phases; and
correlate the one or more peaks to each of the phases of the gait cycle.

6. The system of statement 5, wherein the at least one phase comprises an early swing phase, a mid-swing phase, and a late swing phase.

7. The system of statement 5, wherein the processor executes the program instructions to automatically:

normalize a magnitude of the one or more peaks of each of the phases into normalized values for each of the phases; and
combine the normalized values for each of the phases into a sum.

8. The system of statement 7, wherein the processor executes the program instructions to automatically qualify the vector sum of the step against a threshold value, whereby a step quality value is generated for the step.

9. The system of statement 8, comprising a communication interface in communication with the processor, wherein the processor executes the program instructions to automatically transmit the step quality value, the vector sum, the normalized values, the pressure waveform, or a combination thereof via the communication interface.

10. The system of statement 4, wherein the processor executes the program instructions to automatically:

normalize the one or more peaks into a normalized value; and
compare the normalized value of the one or more peaks with a socket motion threshold value.

11. The system of statement 10, wherein the one or more peaks are normalized into the normalized value using a pressure statistic of at least a portion of the pressure waveform, and wherein the pressure statistic comprises an average, a median, a mode, a root mean square, or a combination thereof.

12. The system of statement 10, wherein the processor executes the program instructions to automatically communicate an adverse condition when the socket motion threshold value is exceeded.

13. The system of statement 1, comprising an inertial sensing package that monitors motion of the prosthetic socket, wherein the processor executes the program instructions to automatically determine an activity level based upon the motion of the prosthetic socket, wherein the vacuum suspension range is determined based in part upon the activity level.

14. The system of statement 13, wherein the inertial sensing package comprises at least one accelerometer sensor, at least one gyroscopic sensor, at least one force sensor, at least one pressure sensor, or a combination thereof.

15. The system of statement 1, wherein the one or more sensors comprise a motion sensor that directly senses the relative socket motion of the prosthetic socket.

16. The system of statement 15, wherein the motion sensor comprises at least one of a hall sensor, an inductive sensor, an ultrasonic sensor, and a force sensor.

17. A method for intelligent vacuum control, the method comprising:

collecting, at one or more suspension levels, motion data of a prosthetic socket, data indicative of internal pressure of the prosthetic socket and data indicative of relative socket motion of the prosthetic socket with one or more sensors coupled to a prosthetic device;
determining, via the motion data of the prosthetic socket, an activity level associated with the data indicative of the internal pressure of the prosthetic socket and the data indicative of the relative socket motion of the prosthetic socket; and
actuating an electric vacuum pump, automatically with a processor operatively coupled to the electric vacuum pump, to adjust the internal pressure of the prosthetic socket to be within each of the one or more suspension levels, while the motion data of the prosthetic socket, the data indicative of the internal pressure of the prosthetic socket and the data indicative of the relative socket motion of the prosthetic socket are collected.

18. The method of statement 17, comprising:

identifying a step via the motion data of the prosthetic socket;
correlating the step to the data indicative of relative socket motion of the prosthetic socket;
normalizing the data indicative of relative socket motion of the prosthetic socket that is correlated to the step into normalized data;
classifying the step according to the normalized data.

19. The method of statement 17, comprising:

comparing the data indicative of the internal pressure of the prosthetic socket and the data indicative of the relative socket motion of the prosthetic socket to expected level of vacuum data corresponding to the activity level associated with the data indicative of the internal pressure of the prosthetic socket and the data indicative of the relative socket motion of the prosthetic socket, wherein the expected level of vacuum data comprises pressure data and relative socket motion data corresponding to a proper fit; and
evaluating a fit of the prosthetic socket based upon a mismatch of the data indicative of the internal pressure of the prosthetic socket and the data indicative of the relative socket motion of the prosthetic socket compared to the expected level of vacuum data.

20. The method of statement 17, comprising:

providing a graphical user interface upon a display operatively connected to a remote processor driven device, wherein the remote processor driven device is in communication with the processor, and wherein the graphical user interface presents at least one of the motion data of the prosthetic socket, the data indicative of the internal pressure of the prosthetic socket and the data indicative of the relative socket motion of the prosthetic socket;
receiving suspension level input via the graphical user interface; and
changing one or more of the one or more multiple suspension levels based upon the suspension level input.

21. A system for intelligent vacuum control, the system comprising:

a prosthetic socket;
an electric vacuum pump that creates a sub-atmospheric pressure within the prosthetic socket;
a pressure sensor that senses an internal pressure of the prosthetic socket and provides a pressure waveform indicative of relative socket motion of the prosthetic socket;
a non-transient computer readable medium comprising program instructions and
a processor operatively connected to the electric vacuum pump, the one or more sensors, and the non-transient computer readable medium, wherein the processor executes the program instructions to automatically:

detect one or more peaks in the pressure waveform;
quantify the relative socket motion based upon the one or more peaks detected in the pressure waveform; and
compare a magnitude of the one or more peaks that are detected to an acceptable value; and
rate a quality of suspension during a step based upon the magnitude of the one or more peaks and the acceptable value that are compared.

22. The system of statement 21, wherein, the processor executes the program instructions to automatically:

select a vacuum suspension range, wherein the selection is at least partially informed by the quality of the suspension; and
actuate the electric vacuum pump, whereby the internal pressure of the prosthetic socket is adjusted to be within the vacuum suspension range.

23. The system of statement 21, further comprising: a display operatively connected to a remote processor driven device, wherein the remote processor driven device is in communication with the processor, and wherein the remote processor driven device automatically:

receives the quality of the suspension from the processor; and
provides a graphical user interface upon the display, wherein the graphical user interface is configured to evaluate or tune operation of the electric vacuum pump.

24. The method of statement 21, wherein: the processor executes the program instructions to automatically: store the quality of the suspension to the non-transient computer readable medium.

## Claims

**1.** A system for intelligent vacuum control (100, 201, 314), the system (100, 201, 314) comprising:

a prosthetic socket (102);
an electric vacuum pump (104a, 104b) that creates a sub-atmospheric pressure within the prosthetic socket (102);
one or more sensors (208) that sense an internal pressure of the prosthetic socket (102);
a non-transient computer readable medium comprising program instructions and stored stiffness data, wherein the stored stiffness data corresponds to a relationship between pressure variation of the internal pressure of the prosthetic socket (102) and relative socket motion of the prosthetic socket (102); and
a processor (206) operatively connected to the electric vacuum pump (104a, 104b), the one or more sensors (208), and the non-transient computer readable medium, wherein the processor (206) executes the program instructions to automatically:

determine data indicative of actual relative socket motion based on the sensed internal pressure of the prosthetic socket (102);
compare the data indicative of actual relative socket motion and the stored stiffness data;
determine an adjusted vacuum suspension level based upon the comparison of the data indicative of the actual relative socket motion and the stored stiffness data; and
actuate the electric vacuum pump(104a, 104b) to adjust the internal pressure of the prosthetic socket (102) toward the adjusted vacuum suspension level.

**2.** The system of claim 1, wherein the processor (206) is configured to execute the program instructions to:

automatically determine a vacuum suspension range based upon the comparison of the data indicative of the actual relative socket motion and the stored stiffness data; and
actuate the electric vacuum pump (104a, 104b) to adjust the internal pressure of the prosthetic socket to be within the vacuum suspension range.

**3.** The system (100, 210, 314) of claim 2, wherein:

the relationship of the stored stiffness data comprises a first slope of the pressure variation of the internal pressure of the prosthetic socket (102) over the relative socket motion of the prosthetic socket (102);
the relationship of the stored stiffness data comprises a second slope of the pressure variation of the internal pressure of the prosthetic socket (102) over the relative socket motion of the prosthetic socket (102);
the first slope is greater than the second slope; and
the vacuum suspension range corresponds to the first slope.

**4.** The system (100, 210, 314) of claim 1, wherein the relationship between pressure variation of the internal pressure of the prosthetic socket (102) and the relative socket motion of the prosthetic socket (102) is for a single patient at a single activity level.

**5.** The system (100, 210, 314) of claim 1, wherein:

the one or more sensors (108) comprise a pressure sensor (108);
the data indicative of the relative socket motion of the prosthetic socket (102) is a pressure waveform provided by the pressure sensor (108); and
the processor (206) executes the program instructions to automatically:

detect one or more peaks in the pressure waveform; and
quantify the relative socket motion based upon the one or more peaks detected in the pressure waveform.

**6.** The system (100, 210, 314) of claim 5, comprising an inertial sensing package (208) that monitors motion of the prosthetic socket (102), wherein the processor (206) executes the program instructions to automatically:

identify a gait cycle for a step from the motion of the prosthetic socket (102), wherein the gait cycle comprises phases; and
correlate the one or more peaks to each of the phases of the gait cycle.

**7.** The system (100, 210, 314) of claim 6, wherein the at least one phase comprises an early swing phase, a mid-swing phase, and a late swing phase.

**8.** The system (100, 210, 314) of claim 6, wherein the processor (206) executes the program instructions to automatically:

normalize a magnitude of the one or more peaks of each of the phases into normalized values for each of the phases; and
combine the normalized values for each of the phases into a sum.

**9.** The system (100, 210, 314) of claim 8, wherein the processor (206) executes the program instructions to automatically qualify the vector sum of the step against a threshold value, whereby a step quality value is generated for the step.

**10.** The system (100, 210, 314) of claim 9, comprising a communication interface (210) in communication with the processor (206), wherein the processor (206) executes the program instructions to automatically transmit the step quality value, the vector sum, the normalized values, the pressure waveform, or a combination thereof via the communication interface (210).

**11.** The system (100, 210, 314) of claim 5, wherein the processor (210) executes the program instructions to automatically:

normalize the one or more peaks into a normalized value; and
compare the normalized value of the one or more peaks with a socket motion threshold value.

**12.** The system (100, 210, 314) of claim 11, wherein the one or more peaks are normalized into the normalized value using a pressure statistic of at least a portion of the pressure waveform.

**13.** The system (100, 210, 314) of claim 12, wherein the pressure statistic comprises an average, a median, a mode, a root mean square, or a combination thereof.

**14.** The system (100, 210, 314) of claim 11, wherein the processor (102) executes the program instructions to automatically communicate an adverse condition when the socket motion threshold value is exceeded.

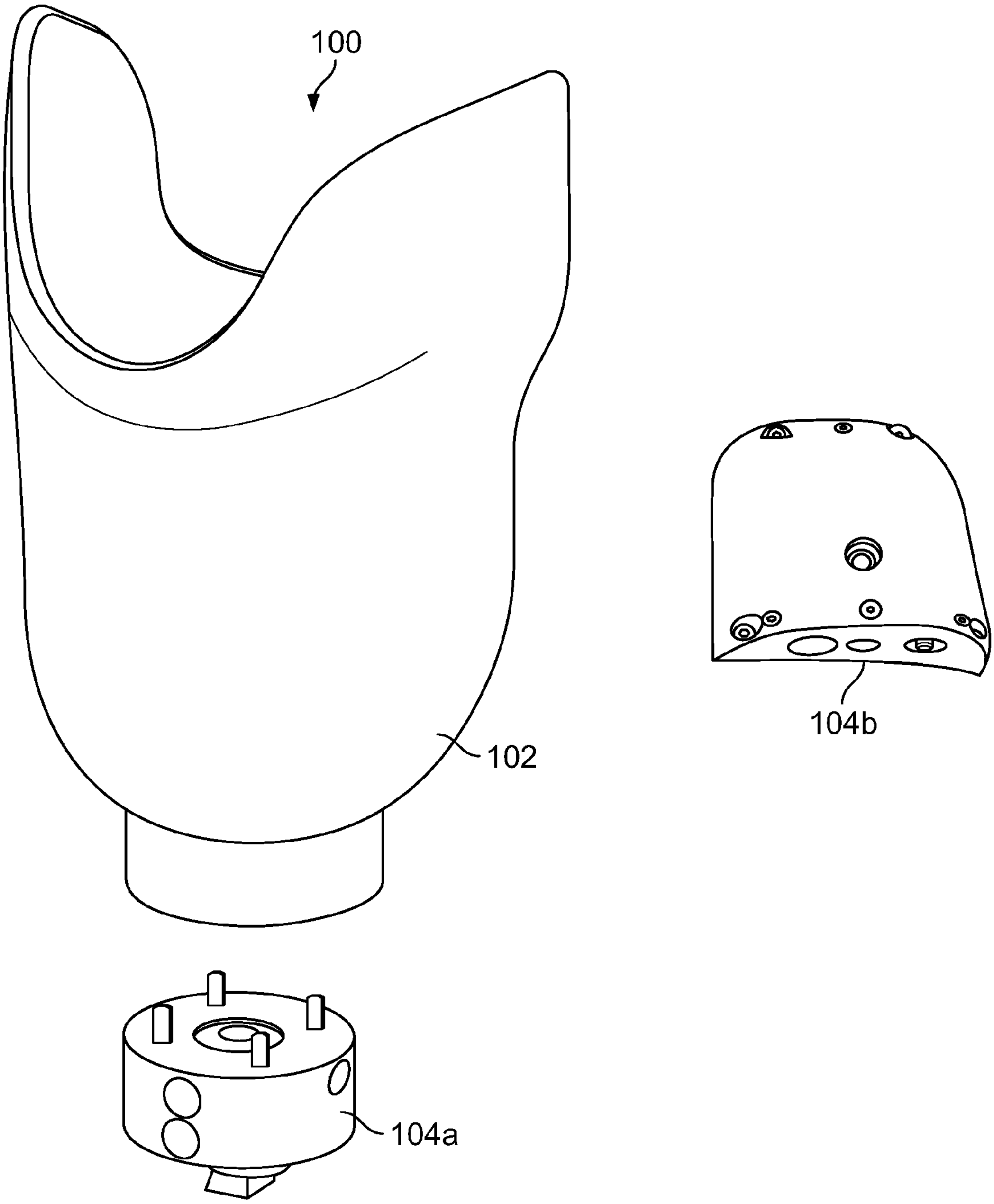


FIG. 1

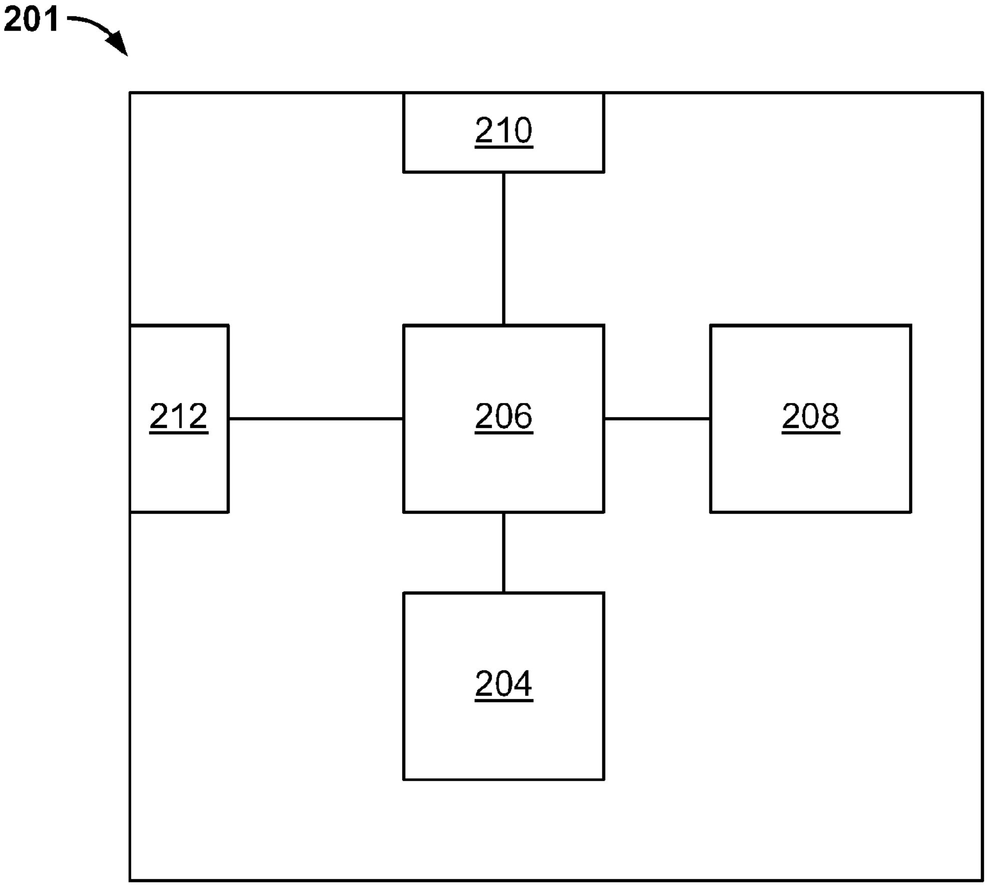


FIG. 2

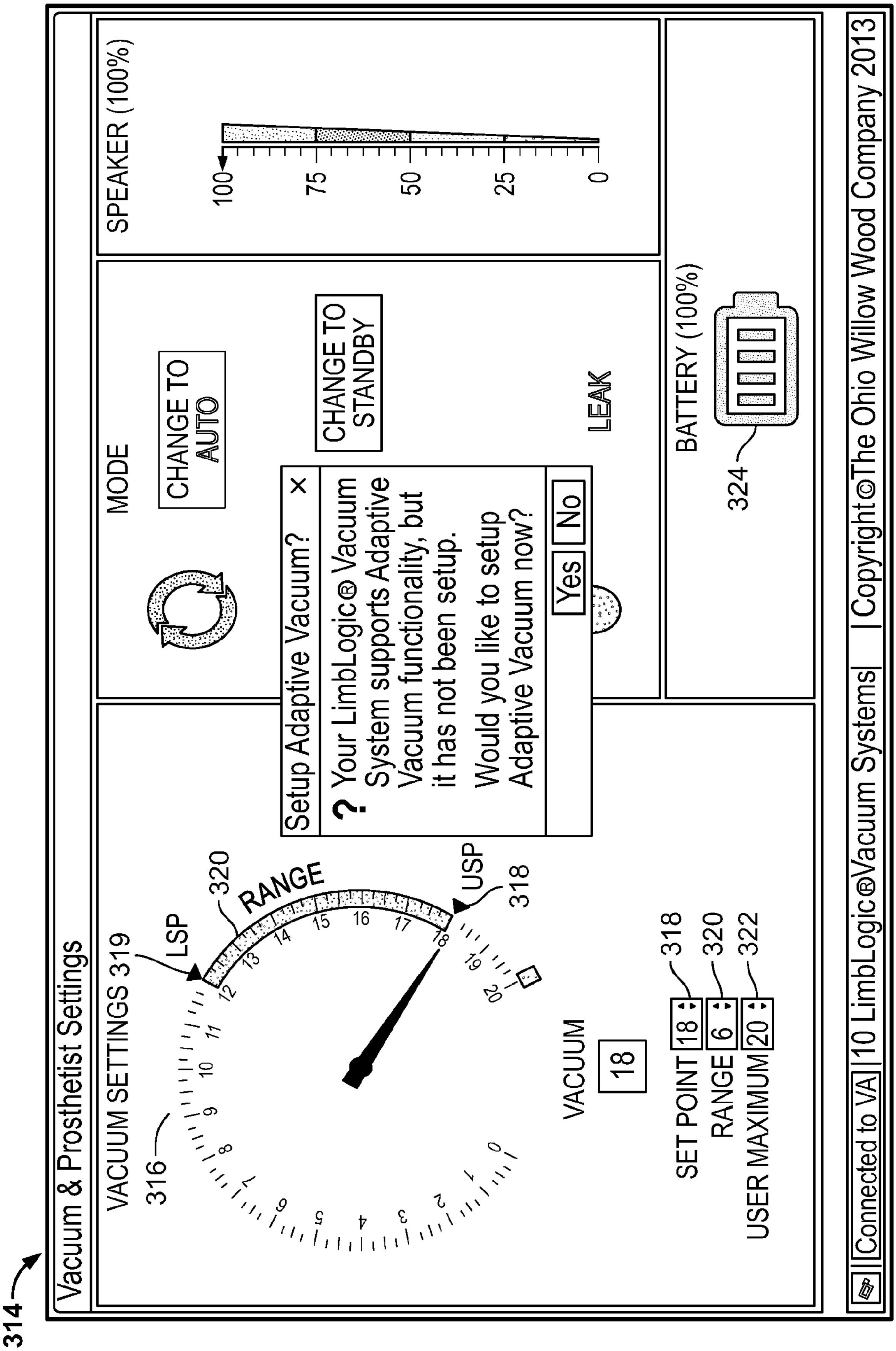
314
Vacuum & Prosthetist Settings
VACUUM SETTINGS 319
316
LSP
320
RANGE
USP
318
VACUUM
18
SET POINT 18
RANGE 6
USER MAXIMUM 20
318
320
322
MODE
CHANGE TO AUTO
CHANGE TO STANDBY
LEAK
Setup Adaptive Vacuum?
Your LimbLogic® Vacuum System supports Adaptive Vacuum functionality, but it has not been setup.
Would you like to setup Adaptive Vacuum now?
Yes
No
SPEAKER (100%)
100
75
50
25
0
BATTERY (100%)
324
Connected to VA
10 LimbLogic® Vacuum Systems
Copyright © The Ohio Willow Wood Company 2013

FIG. 3

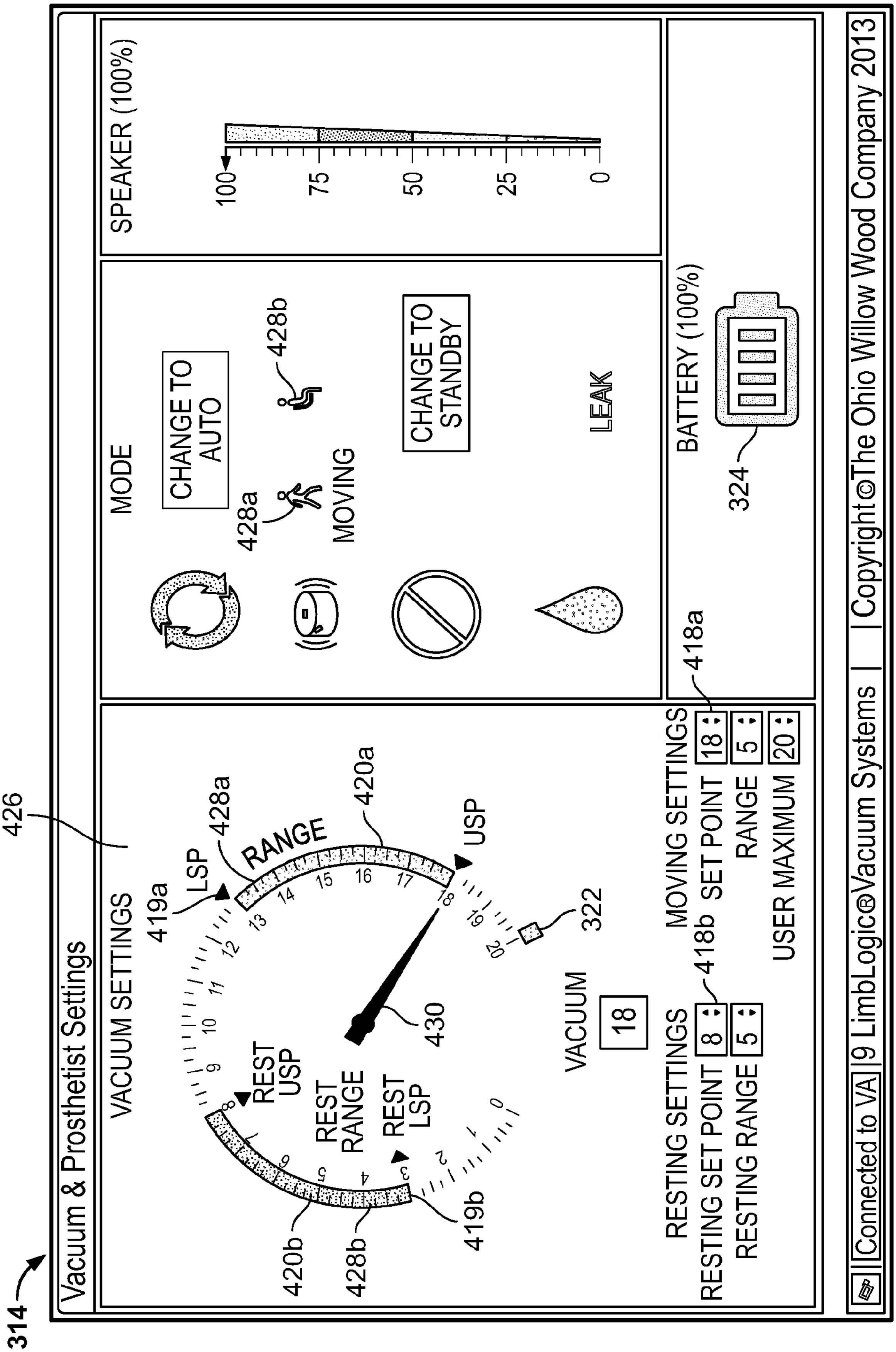
314
Vacuum & Prosthetist Settings
VACUUM SETTINGS
426
419a
LSP
428a
RANGE
420a
USP
322
430
REST USP
REST RANGE
REST LSP
420b
428b
419b
VACUUM
18
RESTING SETTINGS
RESTING SET POINT 8
RESTING RANGE 5
418b
MOVING SETTINGS
SET POINT 18
RANGE 5
USER MAXIMUM 20
418a
MODE
CHANGE TO AUTO
428a
MOVING
428b
CHANGE TO STANDBY
LEAK
BATTERY (100%)
324
SPEAKER (100%)
100
75
50
25
0
Connected to VA
9 LimbLogic®Vacuum Systems
Copyright©The Ohio Willow Wood Company 2013

FIG. 4

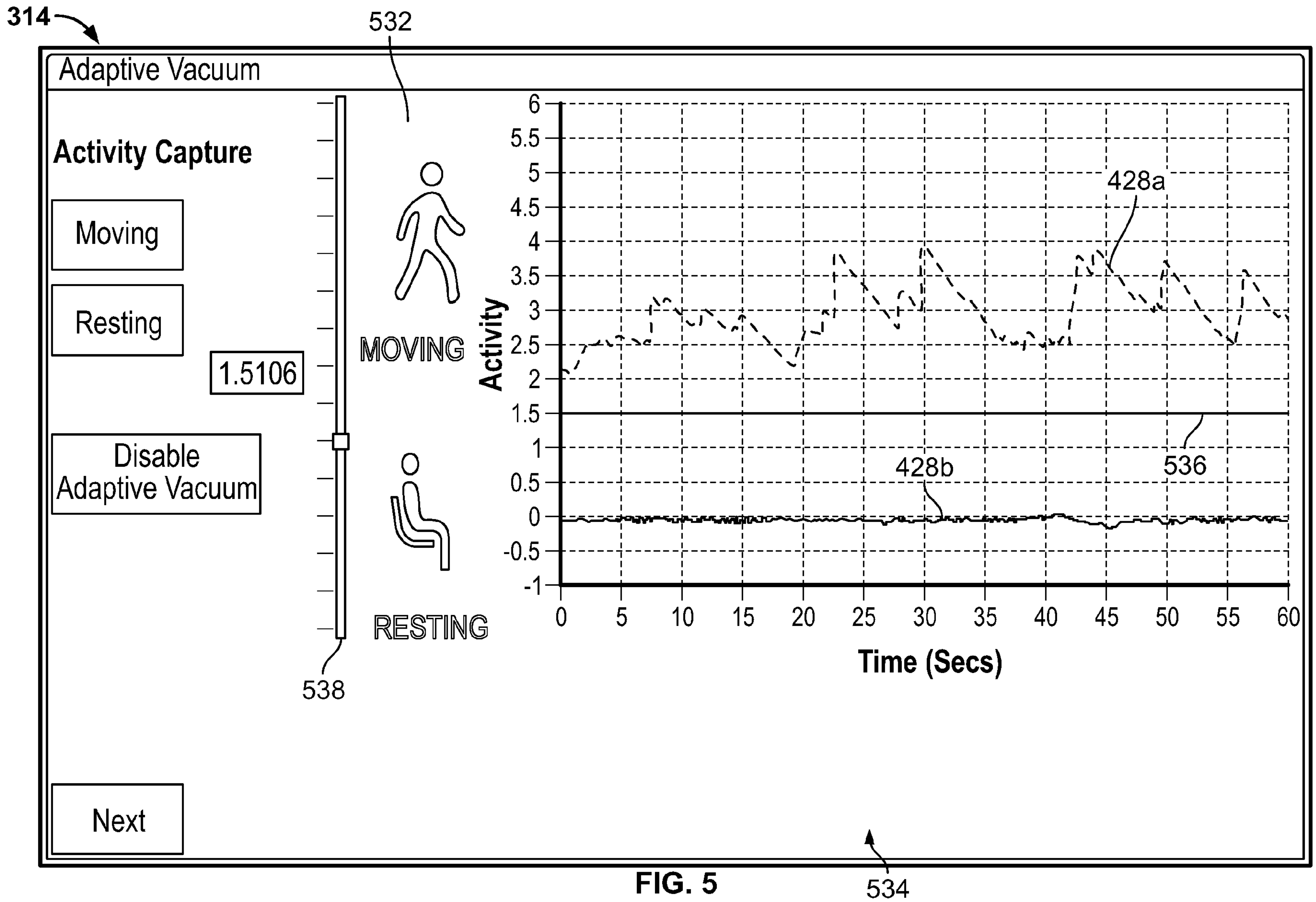

314
532
Adaptive Vacuum
Activity Capture
Moving
Resting
1.5106
Disable
Adaptive Vacuum
Next
MOVING
RESTING
538
Activity
6
5.5
5
4.5
4
3.5
3
2.5
2
1.5
1
0.5
0
-0.5
-1
0
5
10
15
20
25
30
35
40
45
50
55
60
Time (Secs)
428a
428b
536
534


FIG. 5

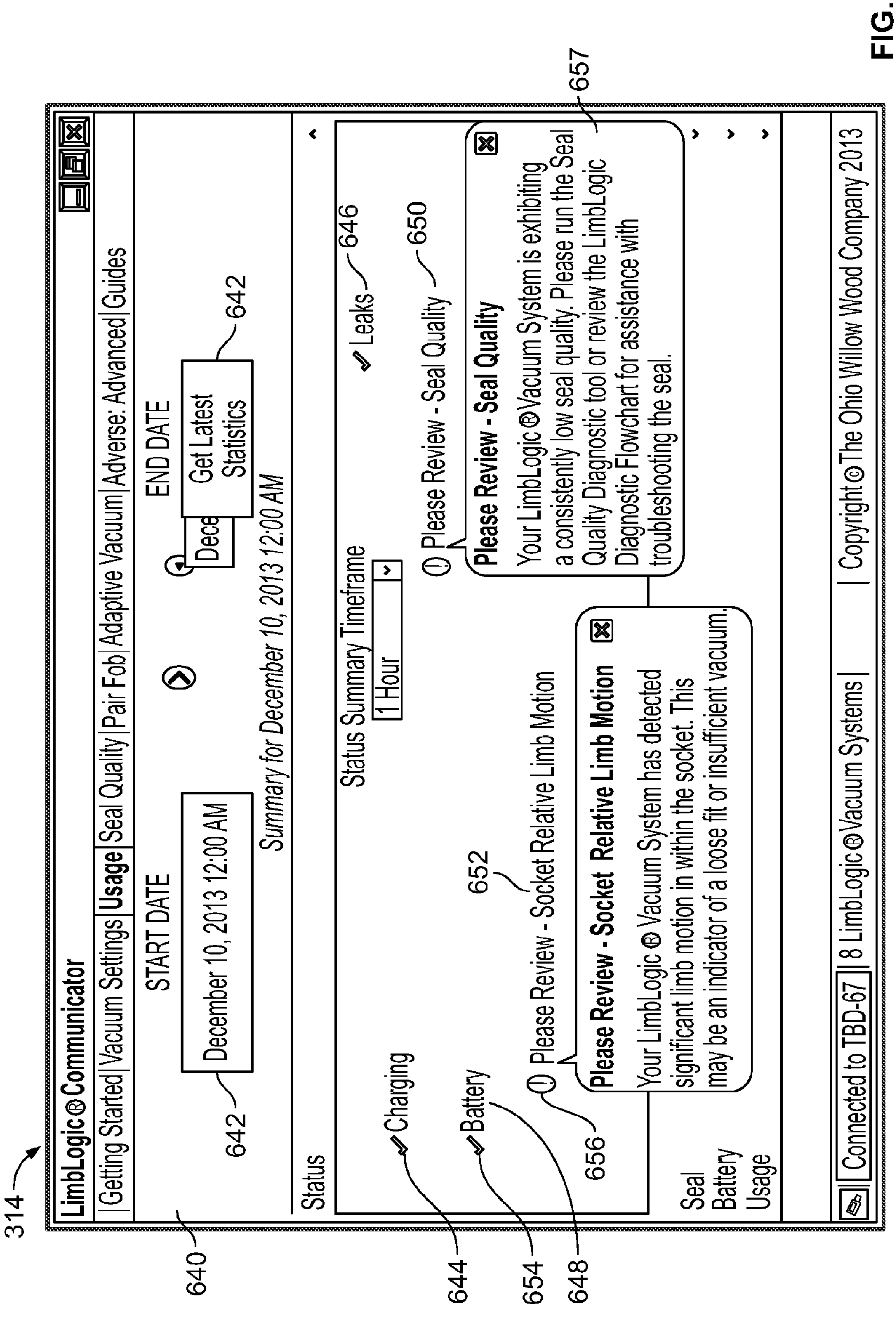
314
640
644
654
648
642
646
650
652
656
657
LimbLogic® Communicator
Getting Started | Vacuum Settings | Usage | Seal Quality | Pair Fob | Adaptive Vacuum | Adverse: Advanced | Guides
START DATE
December 10, 2013 12:00 AM
END DATE
Dece
Get Latest Statistics
Summary for December 10, 2013 12:00 AM
Status
Status Summary Timeframe
1 Hour
Charging
Battery
Leaks
Please Review - Seal Quality
Please Review - Socket Relative Limb Motion
Please Review - Seal Quality
Your LimbLogic® Vacuum System is exhibiting a consistently low seal quality. Please run the Seal Quality Diagnostic tool or review the LimbLogic Diagnostic Flowchart for assistance with troubleshooting the seal.
Please Review - Socket Relative Limb Motion
Your LimbLogic ® Vacuum System has detected significant limb motion in within the socket. This may be an indicator of a loose fit or insufficient vacuum.
Seal
Battery
Usage
Connected to TBD-67
8 LimbLogic® Vacuum Systems
Copyright © The Ohio Willow Wood Company 2013

FIG. 6

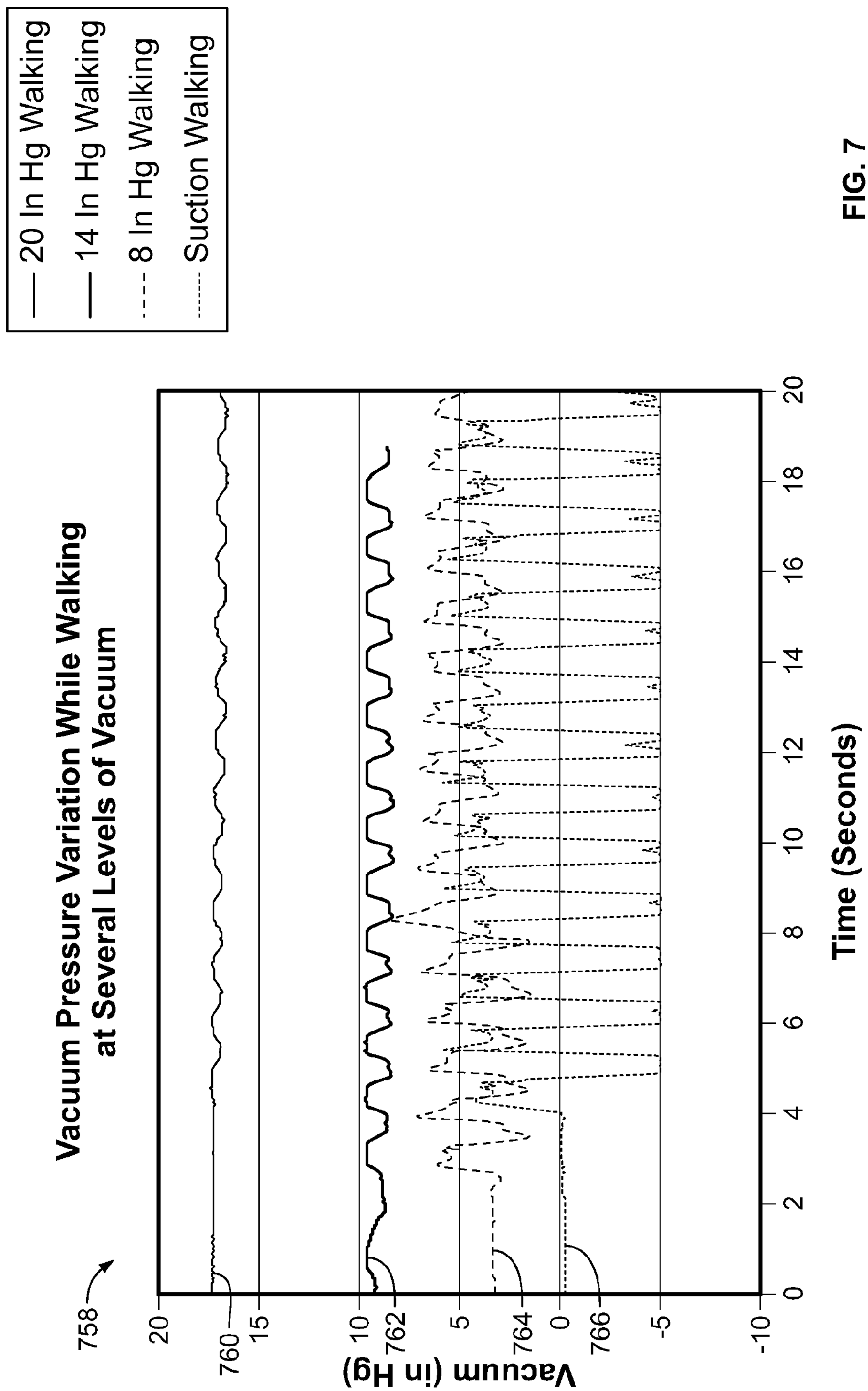

758
Vacuum Pressure Variation While Walking at Several Levels of Vacuum
20 In Hg Walking
14 In Hg Walking
8 In Hg Walking
Suction Walking
Vacuum (in Hg)
20
15
10
5
0
-5
-10
760
762
764
766
Time (Seconds)
0
2
4
6
8
10
12
14
16
18
20


FIG. 7

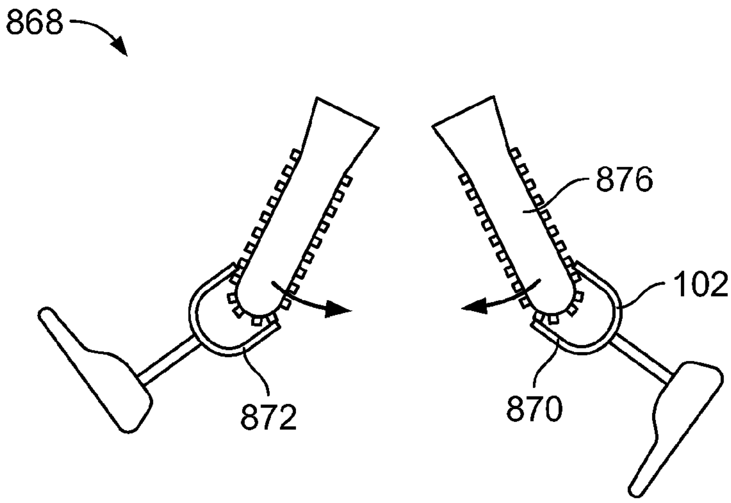


FIG. 8A

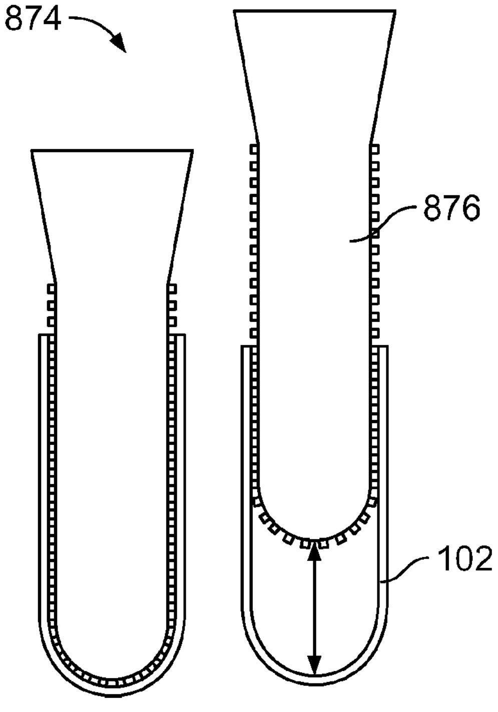


FIG. 8B

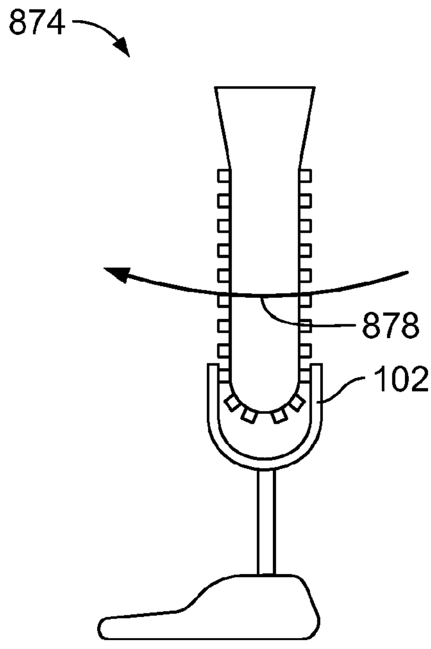


FIG. 8C

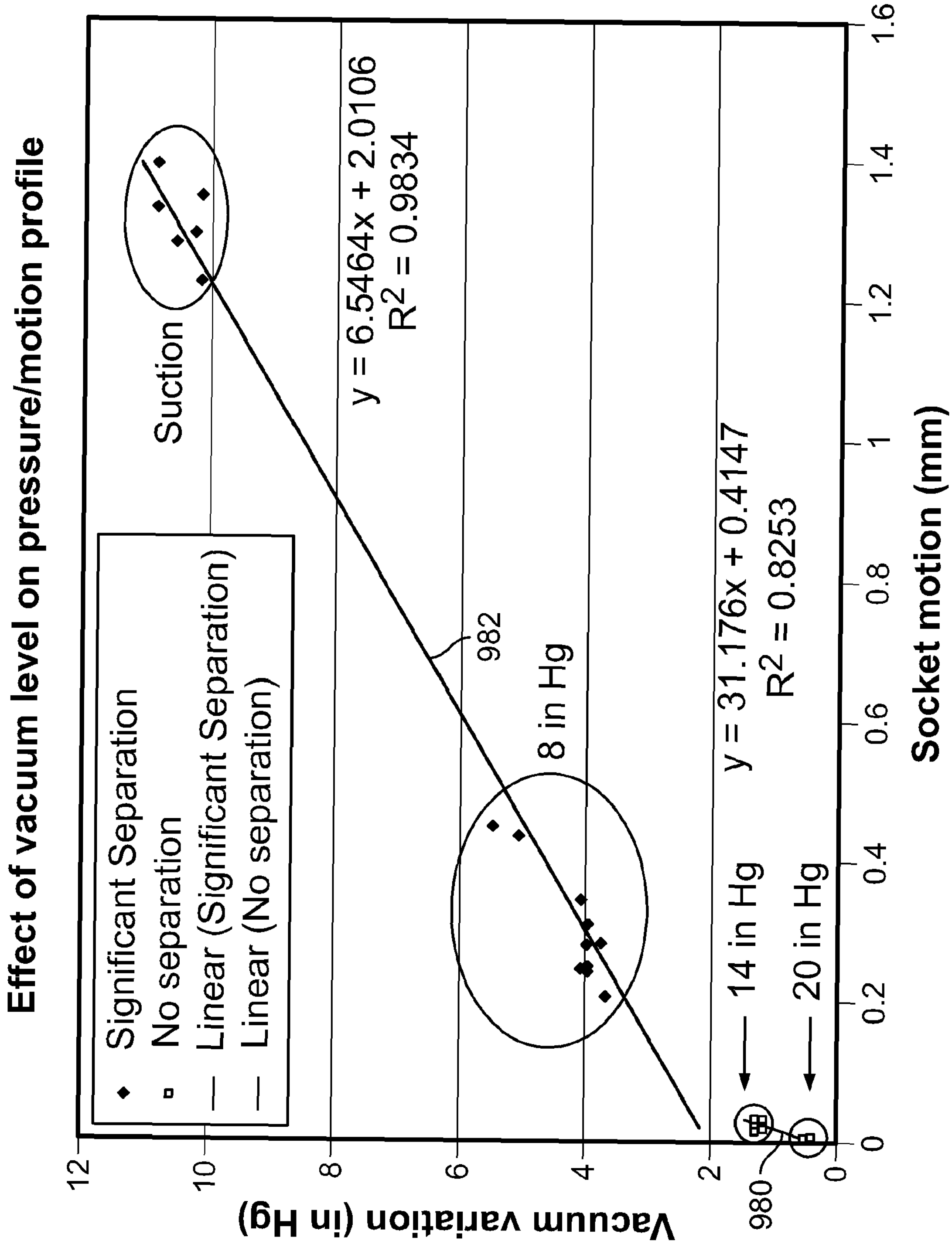
Effect of vacuum level on pressure/motion profile
Significant Separation
No separation
Linear (Significant Separation)
Linear (No separation)
Suction
y = 6.5464x + 2.0106
R2 = 0.9834
982
8 in Hg
y = 31.176x + 0.4147
R2 = 0.8253
14 in Hg
20 in Hg
980
Vacuum variation (in Hg)
Socket motion (mm)
0
2
4
6
8
10
12
0.2
0.4
0.6
0.8
1
1.2
1.4
1.6

FIG. 9

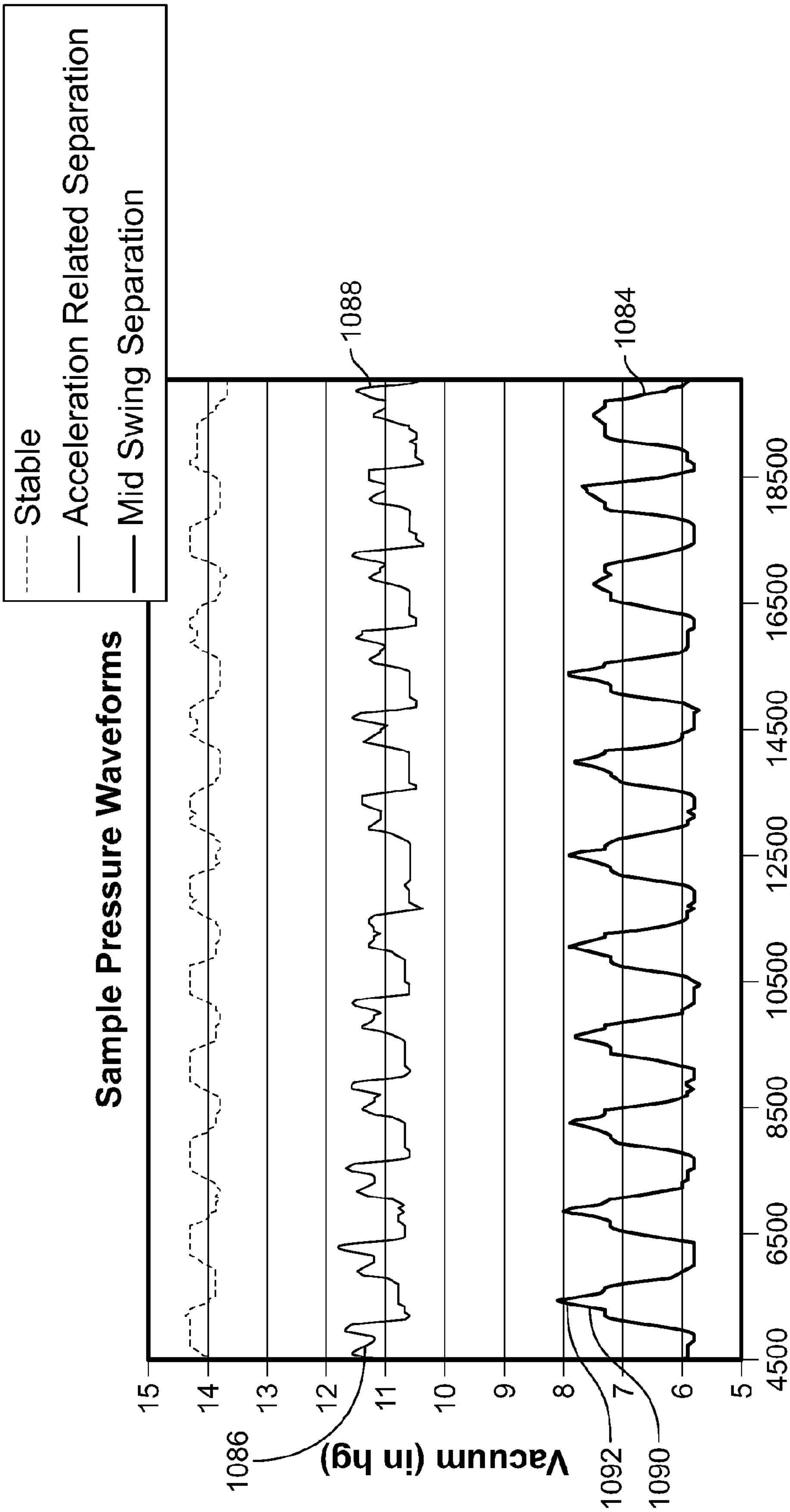
Sample Pressure Waveforms
Stable
Acceleration Related Separation
Mid Swing Separation
Vacuum (in hg)
15
14
13
12
11
10
9
8
7
6
5
Time (ms)
4500
6500
8500
10500
12500
14500
16500
18500
1086
1088
1084
1092
1090

FIG. 10

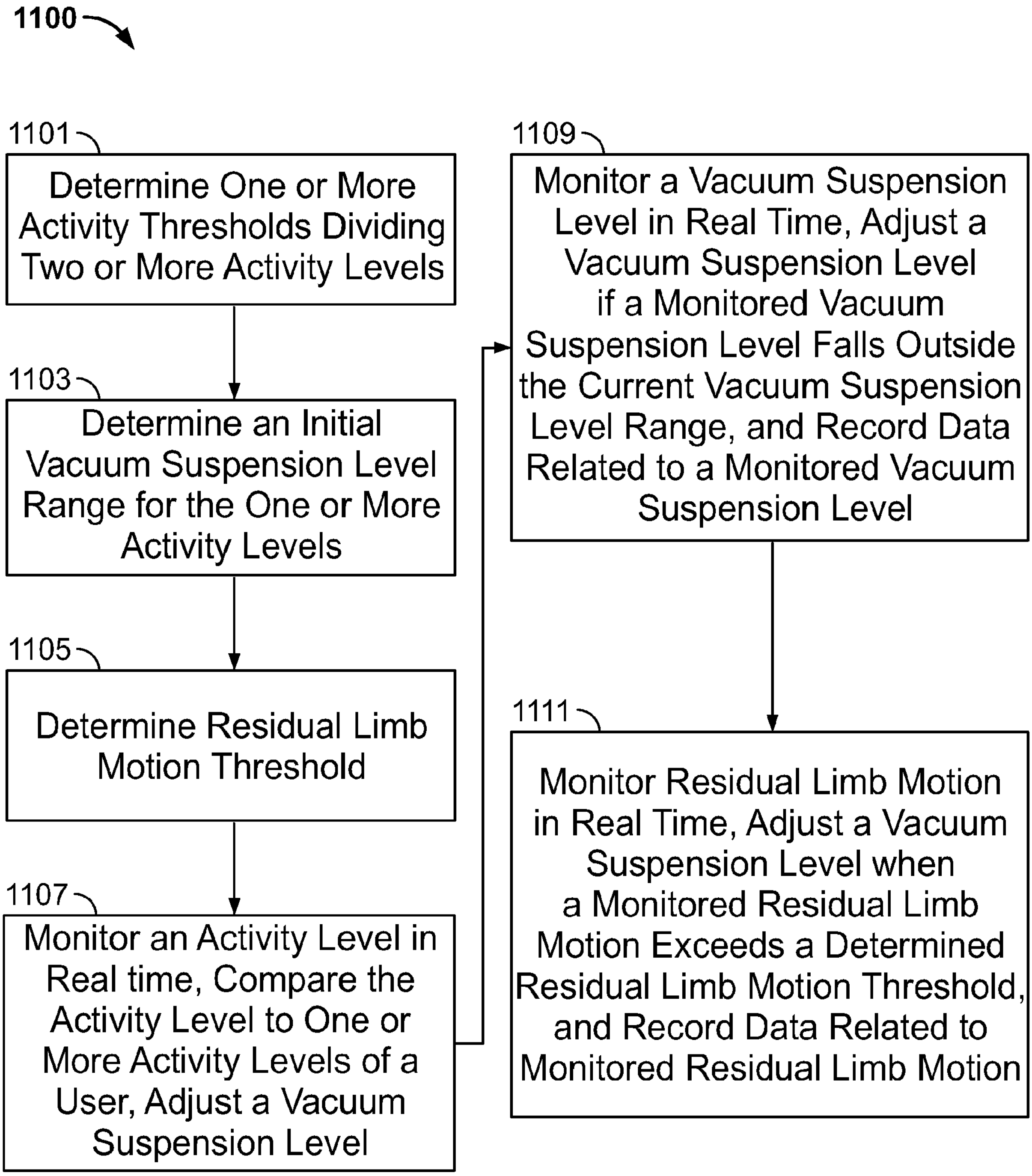
1100
1101
Determine One or More Activity Thresholds Dividing Two or More Activity Levels
1103
Determine an Initial Vacuum Suspension Level Range for the One or More Activity Levels
1105
Determine Residual Limb Motion Threshold
1107
Monitor an Activity Level in Real time, Compare the Activity Level to One or More Activity Levels of a User, Adjust a Vacuum Suspension Level
1109
Monitor a Vacuum Suspension Level in Real Time, Adjust a Vacuum Suspension Level if a Monitored Vacuum Suspension Level Falls Outside the Current Vacuum Suspension Level Range, and Record Data Related to a Monitored Vacuum Suspension Level
1111
Monitor Residual Limb Motion in Real Time, Adjust a Vacuum Suspension Level when a Monitored Residual Limb Motion Exceeds a Determined Residual Limb Motion Threshold, and Record Data Related to Monitored Residual Limb Motion

FIG. 11

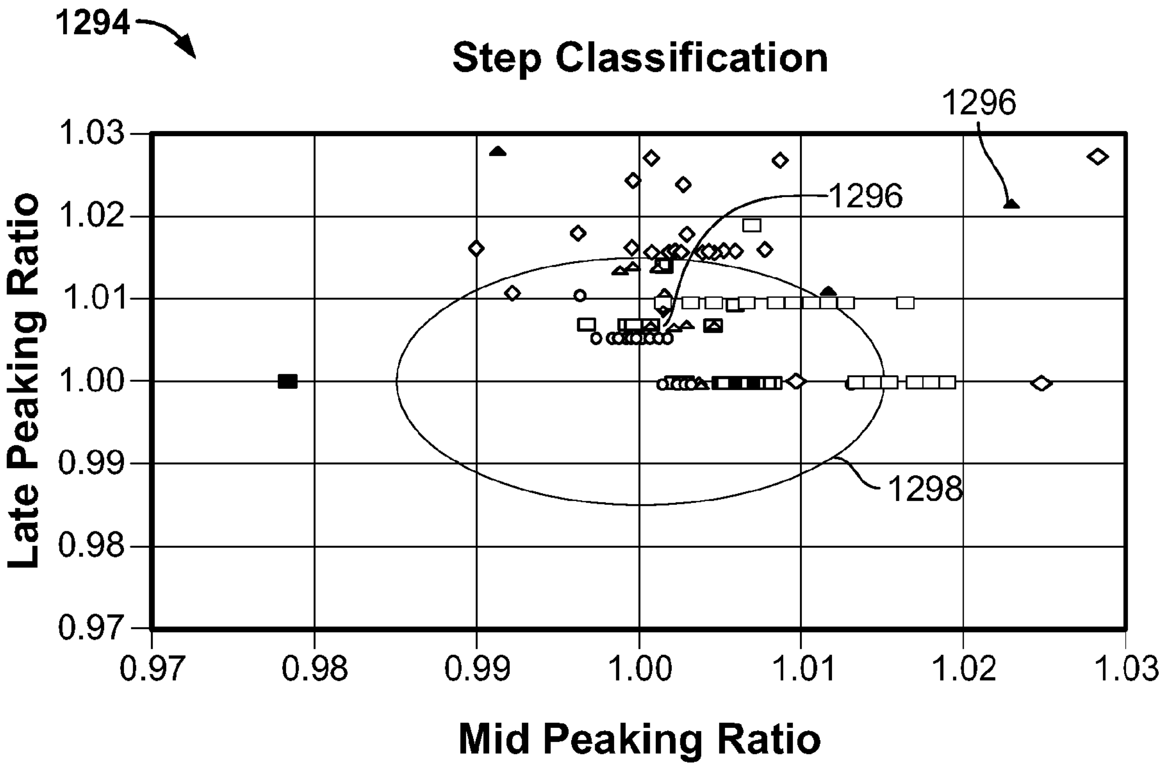


FIG. 12A

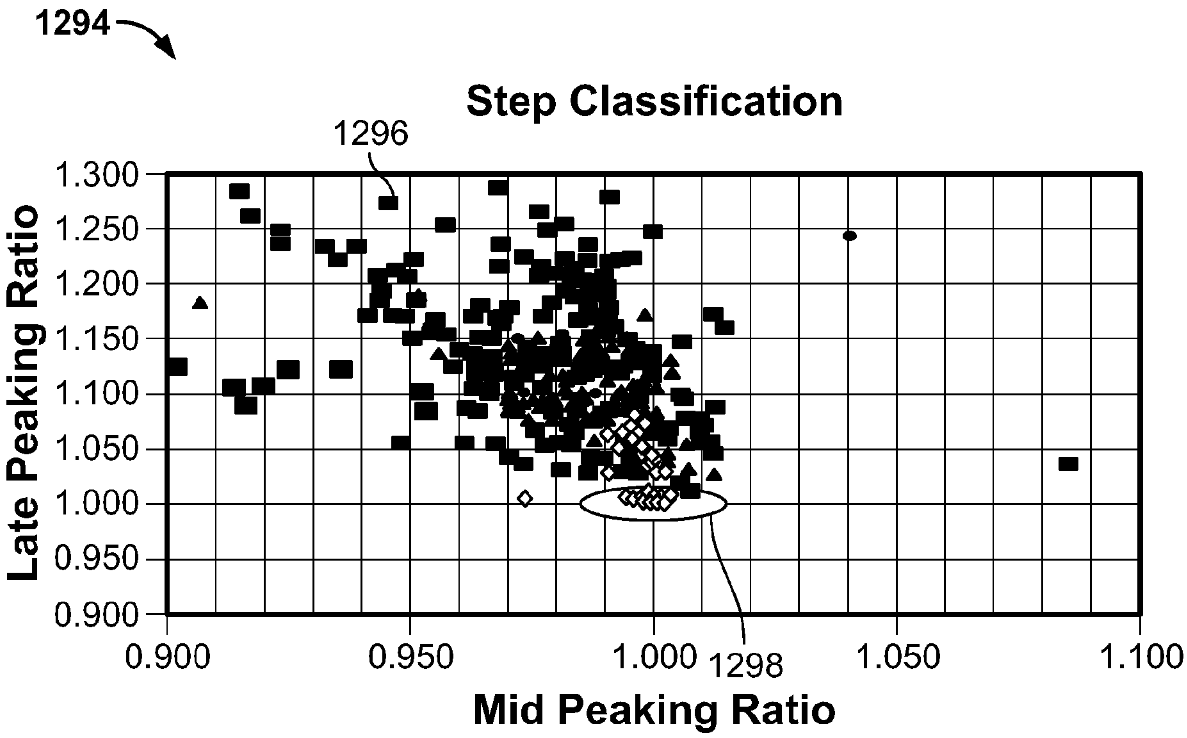


FIG. 12B

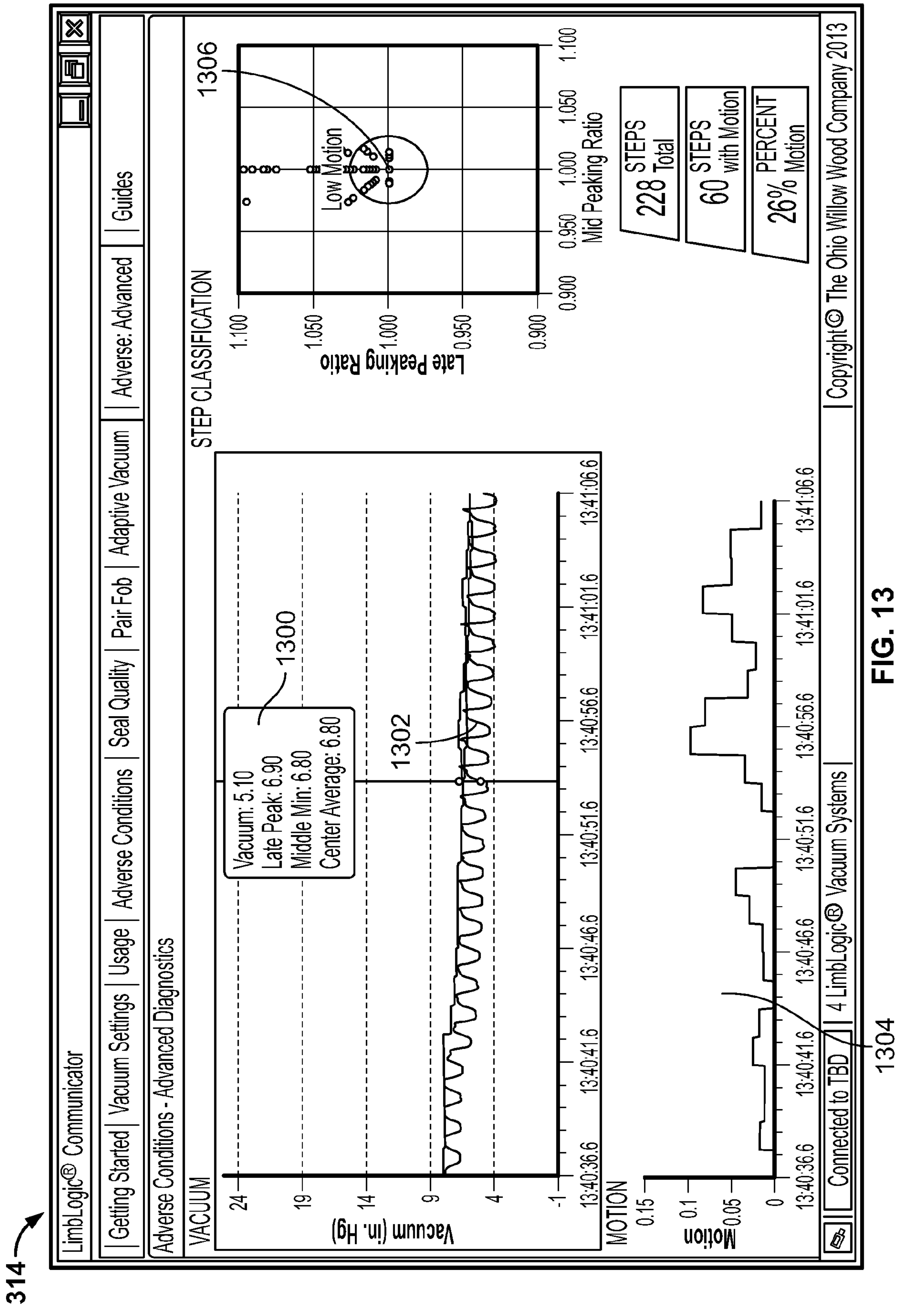
314
LimbLogic® Communicator
Getting Started
Vacuum Settings
Usage
Adverse Conditions
Seal Quality
Pair Fob
Adaptive Vacuum
Adverse: Advanced
Guides
Adverse Conditions - Advanced Diagnostics
VACUUM
Vacuum (in. Hg)
24
19
14
9
4
-1
Vacuum: 5.10
Late Peak: 6.90
Middle Min: 6.80
Center Average: 6.80
1300
1302
13:40:36.6
13:40:41.6
13:40:46.6
13:40:51.6
13:40:56.6
13:41:01.6
13:41:06.6
MOTION
Motion
0.15
0.1
0.05
0
1304
STEP CLASSIFICATION
1306
Low Motion
Late Peaking Ratio
Mid Peaking Ratio
0.900
0.950
1.000
1.050
1.100
228 STEPS Total
60 STEPS with Motion
26% PERCENT Motion
Connected to TBD
4 LimbLogic® Vacuum Systems
Copyright© The Ohio Willow Wood Company 2013

FIG. 13

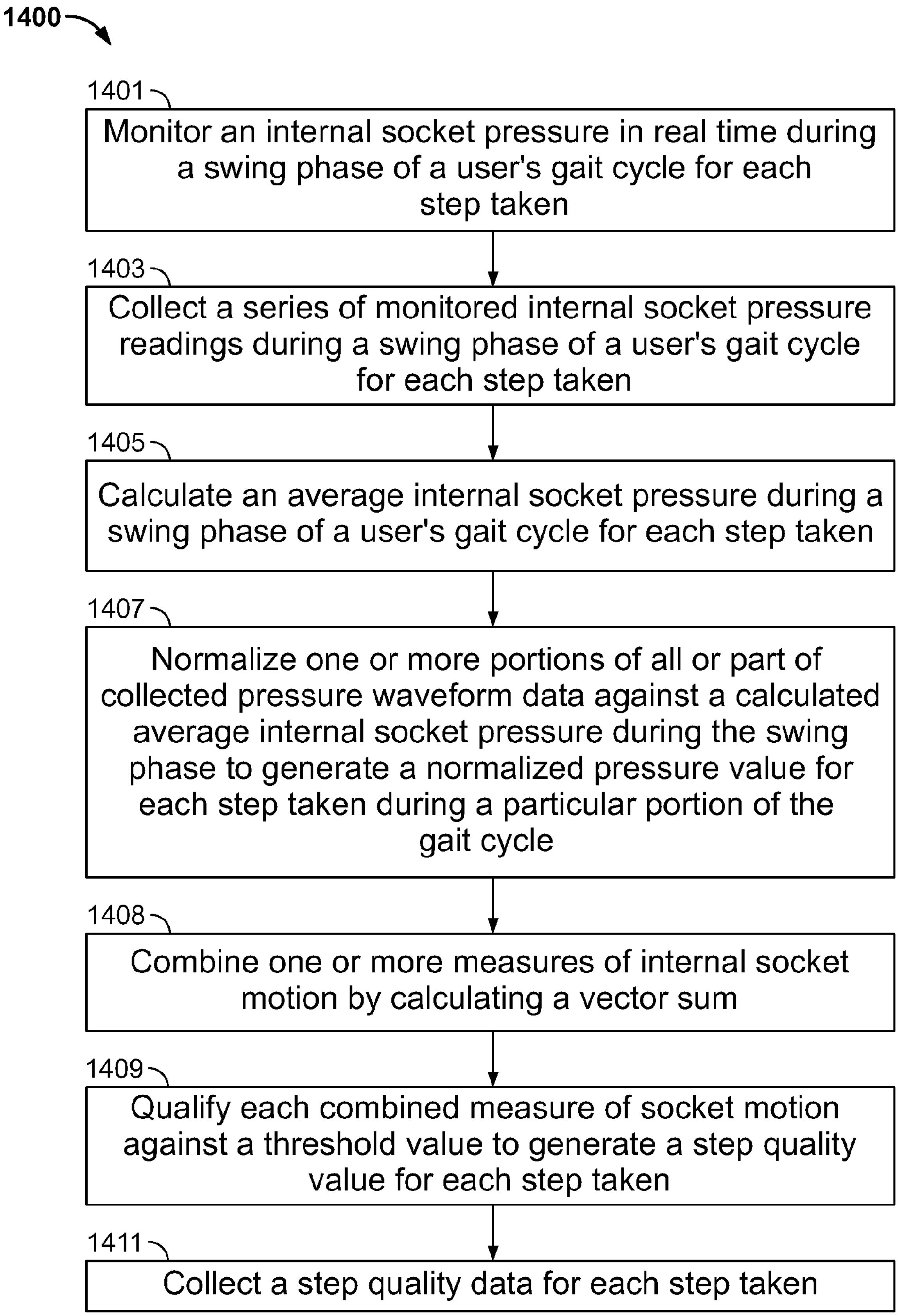
1400
1401
Monitor an internal socket pressure in real time during a swing phase of a user's gait cycle for each step taken
1403
Collect a series of monitored internal socket pressure readings during a swing phase of a user's gait cycle for each step taken
1405
Calculate an average internal socket pressure during a swing phase of a user's gait cycle for each step taken
1407
Normalize one or more portions of all or part of collected pressure waveform data against a calculated average internal socket pressure during the swing phase to generate a normalized pressure value for each step taken during a particular portion of the gait cycle
1408
Combine one or more measures of internal socket motion by calculating a vector sum
1409
Qualify each combined measure of socket motion against a threshold value to generate a step quality value for each step taken
1411
Collect a step quality data for each step taken

FIG. 14

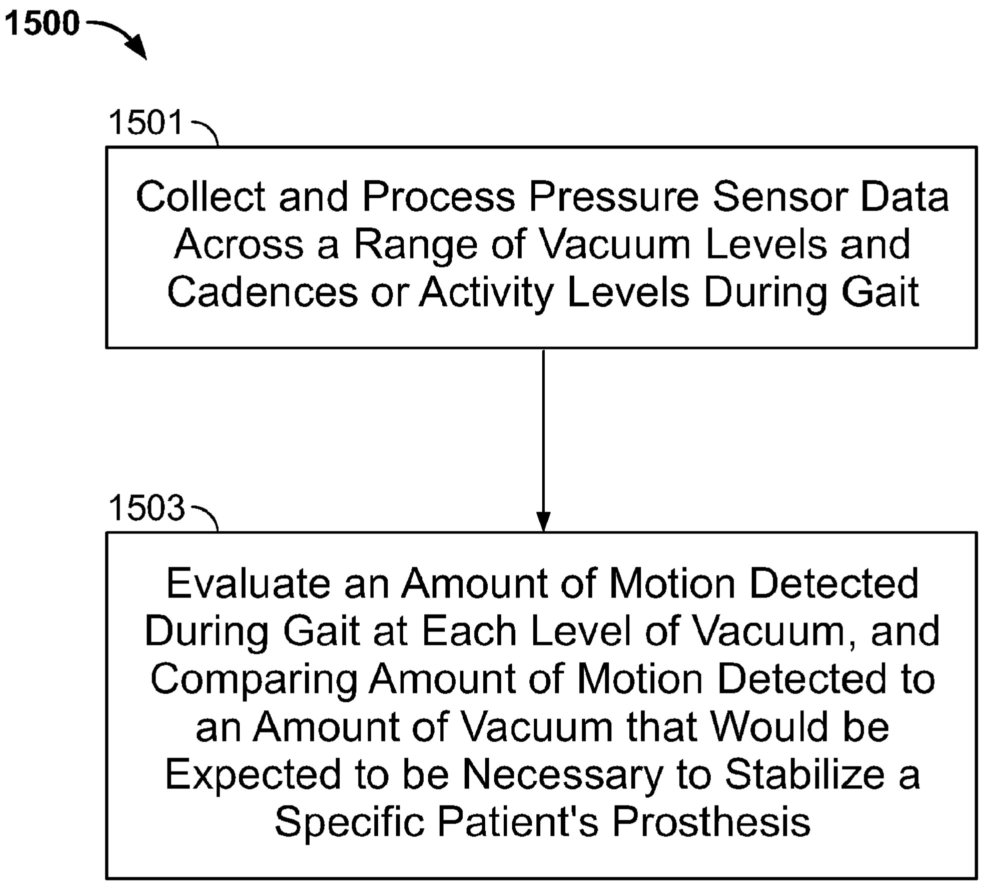
1500
1501
Collect and Process Pressure Sensor Data Across a Range of Vacuum Levels and Cadences or Activity Levels During Gait
1503
Evaluate an Amount of Motion Detected During Gait at Each Level of Vacuum, and Comparing Amount of Motion Detected to an Amount of Vacuum that Would be Expected to be Necessary to Stabilize a Specific Patient's Prosthesis

FIG. 15

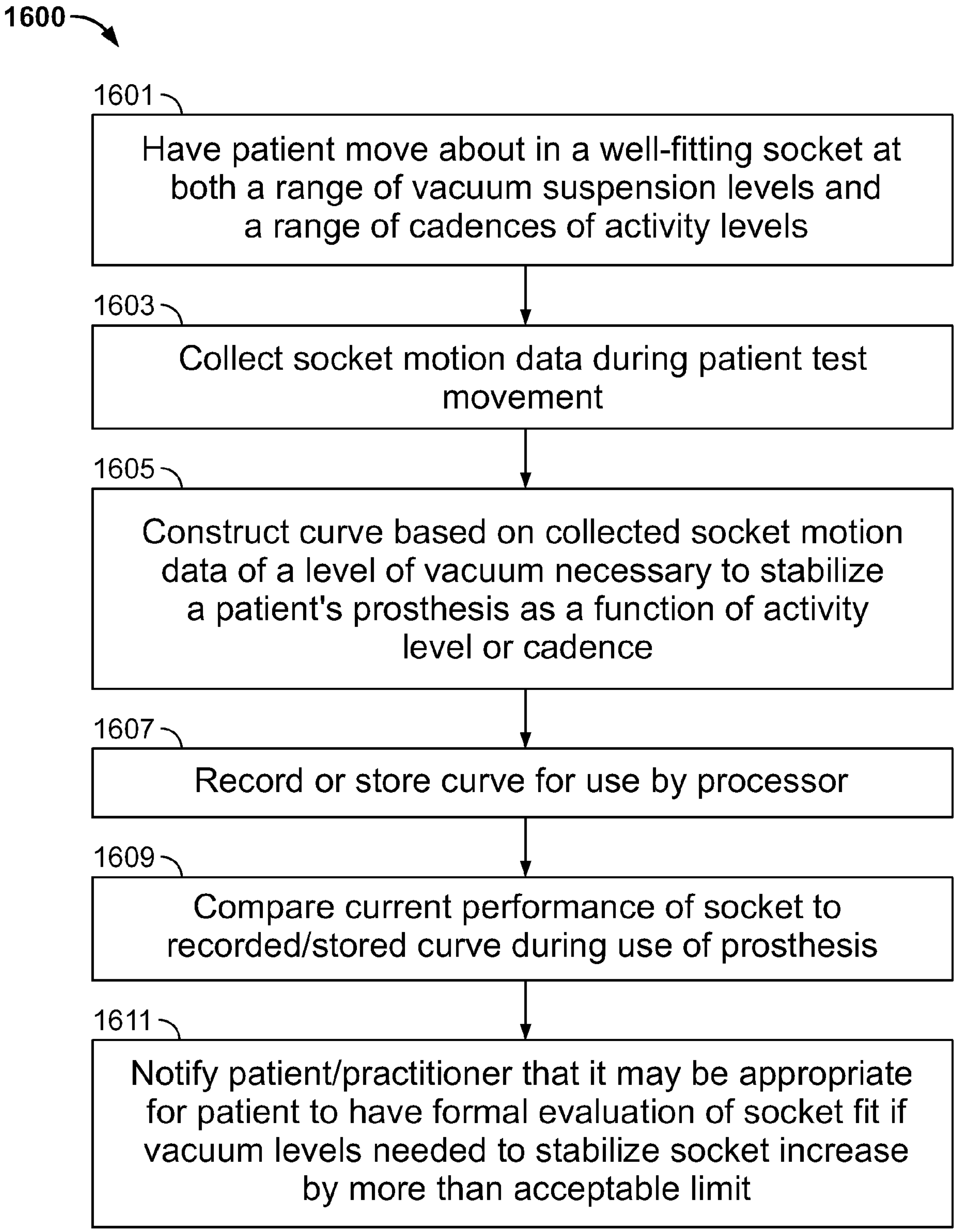
1600
1601
Have patient move about in a well-fitting socket at both a range of vacuum suspension levels and a range of cadences of activity levels
1603
Collect socket motion data during patient test movement
1605
Construct curve based on collected socket motion data of a level of vacuum necessary to stabilize a patient's prosthesis as a function of activity level or cadence
1607
Record or store curve for use by processor
1609
Compare current performance of socket to recorded/stored curve during use of prosthesis
1611
Notify patient/practitioner that it may be appropriate for patient to have formal evaluation of socket fit if vacuum levels needed to stabilize socket increase by more than acceptable limit

FIG. 16

Europäisches Patentamt
European Patent Office
Office européen des brevets

# EUROPEAN SEARCH REPORT

Application Number
EP 19 16 6878

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2007/191965 A1 (COLVIN JAMES M [US] ET AL) 16 August 2007 (2007-08-16)<br>* figures 1-3 *<br>----- | 1-14 | INV.<br>A61F2/68<br>A61F2/70<br>A61F2/80 |
| A | US 2008/243266 A1 (HAYNES MICHAEL L [US] ET AL) 2 October 2008 (2008-10-02)<br>* figure 3 *<br>----- | 1 | |

TECHNICAL FIELDS SEARCHED (IPC)
A61F

The present search report has been drawn up for all claims

1

| Place of search | Date of completion of the search | Examiner |
|---|---|---|
| Munich | 30 July 2019 | Fernández Arillo, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 19 16 6878

This annex lists the patent family members relating to the patent documents cited in the above-mentioned European search report. The members are as contained in the European Patent Office EDP file on
The European Patent Office is in no way liable for these particulars which are merely given for the purpose of information.

30-07-2019

| Patent document cited in search report | Publication date | Patent family member(s) | Publication date |
|---|---|---|---|
| US 2007191965 A1 | 16-08-2007 | CA 2682201 A1 | 25-09-2008 |
| | | EP 2131793 A2 | 16-12-2009 |
| | | US 2007191965 A1 | 16-08-2007 |
| | | US 2012004737 A1 | 05-01-2012 |
| | | US 2016242939 A1 | 25-08-2016 |
| | | WO 2008116051 A2 | 25-09-2008 |
| US 2008243266 A1 | 02-10-2008 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62044924 A
- US 7914586 B
- US 7947085 B
- US 8016892 B
- US 20080243266 A
- US 20120004737 A

### Non-patent literature cited in the description

- **BRYAN A. GARNER.** A Dictionary of Modern Legal Usage. 1995, vol. 624